(19) Europäisches Patentamt
European Patent Office
Office européen des brevets

(11) Numéro de publication : **0 514 273 A1**

(12) # DEMANDE DE BREVET EUROPEEN

(21) Numéro de dépôt : **92401329.5**

(51) Int. Cl.[5] : **C07D 209/44,** A61K 31/40

(22) Date de dépôt : **15.05.92**

(30) Priorité : **17.05.91 FR 9106035**

(43) Date de publication de la demande :
**19.11.92 Bulletin 92/47**

(84) Etats contractants désignés :
**PT**

(71) Demandeur : **RHONE-POULENC RORER SA**
**20, avenue Raymond Aron**
**F-92160 Antony (FR)**

(72) Inventeur : **Achard, Daniel**
**26 rue Adrien Tessier**
**F-94 320 Thiais (FR)**
Inventeur : **Grisoni, Serge**
**17 rue Babeuf**
**F-94 600 Choisy Le Roi (FR)**
Inventeur : **Hanessian, Stephen**
**65 Gables Court**
**Beacons Fields, Quebec, H9W 5H3 (CA)**
Inventeur : **Moutonnier, Claude**
**3 rue Auguste Rodin**
**F-92 350 Le Plessis Robinson (FR)**
Inventeur : **Peyronel, Jean-François**
**6 Parc d'Ardenay**
**F-91 120 Palaiseau (FR)**
Inventeur : **Tabart, Michel**
**Tour Athènes, 75 rue du Javelot**
**F-75 013 Paris (FR)**
Inventeur : **Truchon, Alain**
**73 rue Henri Gorjus**
**F-69 004 Lyon (FR)**

(74) Mandataire : **Savina, Jacques et al**
**RHONE-POULENC RORER S.A., Direction des Brevets, 20 avenue Raymond Aron**
**F-92160 Antony Cédex (FR)**

(54) **Nouveaux dérivés de perhydroisoindole, leur préparation et les compositions pharmaceutiques qui les contiennent.**

(57) Nouveaux dérivés de perhydroisoindole de formule générale (I) dans laquelle les radicaux R sont des atomes d'hydrogène ou forment ensemble une liaison, les symboles R′ sont des radicaux phényle pouvant être substitués par un atome d'halogène ou un radical méthyle en position 2 ou 3, X est un atome d'oxygène ou un radical NH, $R_1$ est phényle éventuellement substitué, cyclohexadiényle, naphtyle, ou hétérocyclyle, $R_2$ est H, halogène, OH, alcoyle, aminoalcoyle, alcoylaminoalcoyle, dialcoylaminoalcoyle, alcoyloxy, alcoylthio, acyloxy, carboxy, alcoyloxycarbonyle éventuellement substitué, benzyloxycarbonyle, amino ou acylamino, $R_3$ est halogène ou OH et $R_4$ est H ou halogène si $R_3$ est halogène sous leurs formes isomères ou leurs mélanges, éventuellement leurs sels lorsqu'ils existent et leur préparation.

Les nouveaux dérivés selon l'invention sont particulièrement intéressants comme antagonistes de la substance P.

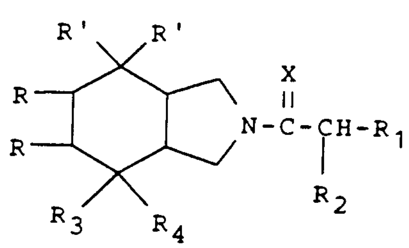
(I)

EP 0 514 273 A1

La présente invention concerne de nouveaux dérivés du perhydroisoindole de formule générale :

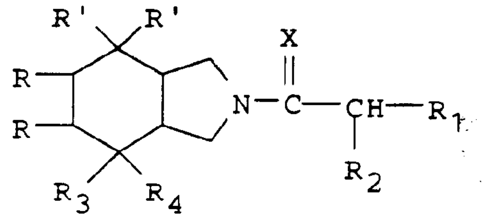

(I)

ainsi que leurs sels lorsqu'ils existent, qui antagonisent les effets de la substance P et sont de ce fait particulièrement intéressants dans les domaines thérapeutiques où cette substance est connue pour intervenir.

Dans le brevet américain 4 042 707 avaient été décrits des produits dérivés de l'isoindole de formule générale:

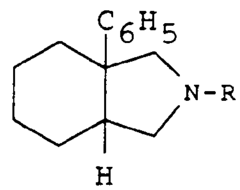

ayant une activité opiacée.

Ces produits n'ont pas d'activité vis à vis de la substance P.

Jusqu'à présent, malgré les recherches mises en oeuvre et malgré l'intérêt suscité [M.R. Hanley, TINS,(5) 139 (1982)], il n'avait pratiquement pas été découvert de produit agissant spécifiquement sur la substance P et ayant une structure non peptidique, c'est pourquoi les dérivés de l'isoindole de formule générale (I) présentent un intérêt considérable.

Dans la formule générale (I) :

- les radicaux R sont identiques et représentent des atomes d'hydrogène ou forment ensemble une liaison,
- les symboles R' sont identiques et représentent des radicaux phényle éventuellement substitués par un atome d'halogène ou par un radical méthyle en position 2 ou 3,
- le symbole X représente un atome d'oxygène, ou un radical NH,
- le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués (par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,
- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,
- le symbole $R_3$ représente un atome d'halogène ou un radical hydroxy et
- le symbole $R_4$ représente un atome d'hydrogène ou simultanément à $R_3$ représente un atome d'halogène.

Il est entendu que les radicaux alcoyle ou acyle cités ci-dessus contiennent 1 à 4 atomes de carbone en chaîne droite ou ramifiée.

Lorsque R' porte un substituant halogène, ce dernier peut être choisi parmi le chlore ou le fluor.

Lorsque $R_1$ contient un atome d'halogène, ce dernier peut être choisi parmi le chlore, le brome, le fluor ou l'iode.

Lorsque $R_1$ représente un radical hétérocyclyle mono ou polycyclique, saturé ou insaturé, à titre d'exemple il peut être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle.

Lorsque $R_1$ représente phényle substitué par une chaîne portant un hétérocycle, ce dernier peut être choisi parmi pyrrolidinyle, morpholino, pipéridinyle, tétrahydropyridinyle pipérazinyle, thiomorpholino.

Lorsque $R_3$ est un atome d'halogène , il peut être avantageusement choisi parmi le fluor ou le chlore.

Par ailleurs, les produits de formule générale (I) présentent différentes formes stéréoisomères, il est entendu que les dérivés de l'isoindole de forme (3aR,7aR) à l'état pur, ou sous forme de mélange des formes cis (3aRS,7aRS), entrent dans le cadre de la présente invention. Lorsque les radicaux $R_3$ et $R_4$ sont différents, il est également entendu que le substituant $R_3$ peut être en position axiale ou équatoriale et de ce fait que les dérivés R et S ainsi que leurs mélanges entrent aussi dans le cadre de le présente invention. De plus, lorsque le symbole $R_2$ est autre que l'atome d'hydrogène, la chaîne substituée sur l'isoindole présente un centre chiral, il est entendu que les formes stéréoisomères et leurs mélanges entrent aussi dans le cadre de la présente invention.

Selon l'invention les dérivés du perhydroisoindole de formule générale (I) peuvent être obtenus par action de l'acide de formule générale :

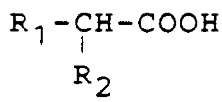

(II)

ou d'un dérivé réactif de cet acide, dans lequel $R_1$ et $R_2$sont définis comme précédemment, sur un dérivé de l'isoindole de formule générale :

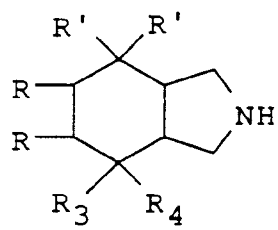

(III)

dans laquelle les symboles R, R', $R_3$ et $R_4$ sont définis comme précédemment suivie le cas échéant de la transformation de l'amide obtenu en une amidine .

Il est entendu que, les radicaux amino, alcoylamino ou carboxy contenus dans $R_1$ et/ou $R_2$ sont de préférence préalablement protégés. La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A. Wiley -Interscience Publication (1981), ou par Mc Omie, Protective Groups in Organic Chemistry, Plenum Press (1973).

A titre d'exemple,

- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque $R_2$ représente un radical hydroxy, il est préférable de protéger préalablement ce radical. La protection s'effectue par exemple par un radical acétyle, trialcoylsilyle, benzyle, ou sous forme d'un carbonate par un radical -COORa dans lequel Ra est un radical alcoyle ou benzyle.

Lorsque l'on effectue la condensation d'un dérivé réactif de l'acide de formule générale (II), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, dinitro-2,4 phényle, pentachlorophényle ou phtalimido.

La réaction s'effectue généralement à une température comprise entre -40 et +40°C, dans un solvant organique tel qu'un solvant chloré (dichlorométhane, dichloréthane, chloroforme par exemple), un hydrocarbure (toluène par exemple), un éther (tétrahydrofuranne, dioxanne par exemple), un ester (acétate d'éthyle par

exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthyl-morpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide], le NN'-carbonyl-diimidazole ou l'éthoxy-2 éthoxycarbonyl-1 dihydro-1,2 quinoléine ou bien en milieu hydroorganique,en présence d'un agent alcalin de condensation comme le bicarbonate de sodium, puis on transforme le cas échéant l'amide obtenu en une amidine telle que définie précédemment.

La transformation de l'amide de formule générale (I) en une amidine pour laquelle X est un radical NH s'effectue en préparant le dérivé de l'isoindolium de formule générale :

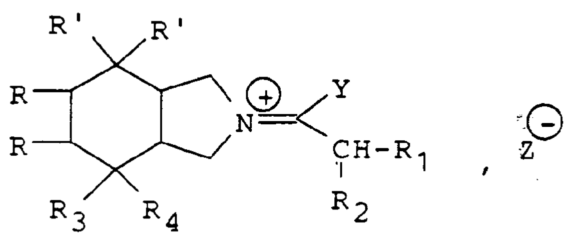

(IV)

dans laquelle R, R', $R_1$, $R_2$, $R_3$ et $R_4$ sont définis comme précédemment, Y représente un atome de chlore, un radical méthoxy ou éthoxy et $Z^-$ représente un ion chlorure, tétrafluoroborate, fluorosulfonate, trifluorométhylsulfonate, méthylsulfate, ou éthylsulfate, puis par action de l'ammoniac sur le dérivé de l'isoindolium.

Il est entendu que, lorsque $R_3$ est hydroxy, Y est autre qu'un atome de chlore.

La préparation du dérivé de l'isoindolium de formule générale (IV) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformate de trichlorométhyle, le tétrafluoroborate de triéthyl (ou de triméthyl) oxonium, le triflate de méthyle (ou d'éthyle), le fluorosulfonate de méthyle (ou d'éthyle) ou le sulfate de méthyle (ou d'éthyle). La réaction s'effectue dans un solvant chloré (dichlorométhane, dichloréthane par exemple) ou dans un hydrocarbure aromatique (toluène par exemple), à une température comprise entre 0°C et la température de reflux du mélange réactionnel. L'action de l'ammoniac sur le dérivé de formule générale (IV) s'effectue dans un solvant organique anhydre tel qu'un solvant chloré (dichlorométhane, dichloréthane par exemple), dans un mélange alcool-solvant chloré, dans un éther (tétrahydrofurane par exemple), dans un ester (par exemple acétate d'éthyle), dans un solvant aromatique (toluène par exemple) ou dans un mélange de ces solvants, à une température comprise entre -20°C et la température de reflux du mélange réactionnel.

Il n'est pas indispensable d'avoir isolé le dérivé de l'isoindolium de formule générale (IV) pour le mettre en oeuvre dans cette réaction.

Selon l'invention le dérivé de l'isoindole de formule générale (I) pour lequel $R_3$ représente un atome d'halogène et $R_4$ représente un atome d'hydrogène peut également être obtenus par halogénation du dérivé correspondant de l'isoindole de formule générale (I) pour lequels $R_3$ est un radical hydroxy, $R_4$ est un atome d'hydrogène et X est un atome d'oxygène, puis le cas échéant transformation l'amide obtenu en une amidine.

Lorsque l'on veut obtenir un produit pour lequel $R_3$ représente un atome de fluor, la réaction s'effectue avantageusement au moyen d'un agent de fluoration comme un fluorure de soufre (trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme l'hexafluoropropyl diéthylamine (brevet japonnais 2 039 546) ou la N-(chloro-2 trifluoro-1,1,2 éthyl) diéthylamine, comme le tétrafluorure de sélenium (J. Am. Chem. Soc., 96,925 (1974) ou comme le tétrafluorophénylphosphorane (Tet. Let., 907 (1973), en opèrant dans un solvant organique tel qu'un solvant chloré (dichlorométhane, dichloréthane par exemple) à une température comprise entre -30 et 30°C. Il est entendu que la mise en oeuvre de l'alcool de configuration (S) conduit au dérivé fluoré de configuration (R) et que la mise en oeuvre de l'alcool de configuration (R) conduit au dérivé fluoré de configuration (S).

Lorsque l'on veut obtenir un produit pour lequel $R_3$ représente un atome de chlore, le dérivé chloré de configuration (R) peut être obtenu par traitement de l'alcool (S) par le pentachlorure de phosphore dans les conditions définies par R.J. Cremlyn et coll., J. Chem. Soc., 3794 (1954) ; le dérivé chloré de configuration (S) peut être obtenu par traitement de l'alcool (S) par le chlorure de thionyle dans les conditions citées par R.J. Cremlyn dans la référence mentionnée ci-dessus.

Selon l'invention les dérivés de l'isoindole de formule générale (I) pour lesquels $R_3$ est un radical hydroxy et $R_4$ est un atome d'hydrogène, peuvent également être obtenus par réduction du dérivé de l'isoindolone de formule générale :

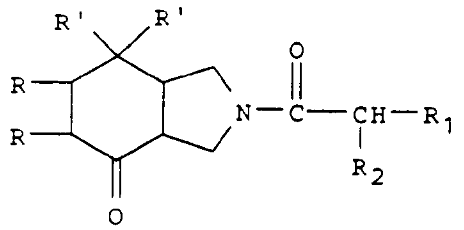

(V)

dans laquelle R, R', $R_1$ et $R_2$ sont définis comme précédemment, suivie de la séparation des isomères axial et équatorial et de la transformation de l'amide obtenu en une amidine.

La réduction s'effectue avantageusement au moyen d'un borohydrure alcalin (borohydrure de sodium, tri-s.butylborohydrure de lithium) dans un solvant tel qu'un alcool (méthanol, éthanol par exemple) ou un éther (tétrahydrofuranne) en milieu basique, à une température comprise entre -20 et 50°C.

Selon l'invention, les dérivés de l'isoindole de formule générale (I) pour lesquels X est un radical imino peuvent également être obtenus à partir du dérivé de l'isoindole de formule générale (III), par action d'un produit de formule générale :

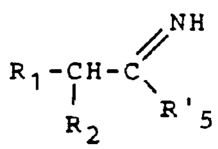

(VI)

éventuellement à l'état de sel, dans laquelle $R_1$ et $R_2$ sont définis comme précédemment et $R'_5$ représente un radical alcoyloxy contenant 1 à 4 atomes de carbone en chaîne droite ou ramifiée ou un radical méthylthio, éthylthio, benzylthio ou alcoyloxycarbonylméthylthio.

La réaction est mise en oeuvre au moyen du dérivé de formule générale (VI) éventuellement préparé in situ, dans un solvant organique tel qu'un solvant chloré (dichlorométhane, dichloréthane par exemple), un éther (tétrahydrofuranne par exemple), un hydrocarbure aromatique (toluène par exemple) ou un nitrile par exemple l'acétonitrile a une température comprise entre 0°C et la température de reflux du mélange réactionnel.

Il est entendu que, dans l'éventualité où les radicaux $R_1$ et/ou $R_2$ du produit de formule générale (VI) portent des substituants pouvant interférer avec la réaction, ces derniers doivent être préalablement protégés.

Les acides de formule générale (II) peuvent être préparés selon les méthodes décrites ci-après dans les exemples, ou par analogie avec ces méthodes.

Le dérivé de l'isoindole de formule générale (III) pour lequel $R_3$ est un atome d'halogène et $R_4$ est un atome d'hydrogène ou d'halogène peut être préparé par halogénation d'un isoindole de formule générale :

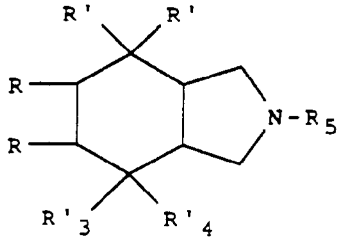

(VII)

dans laquelle R et R' sont définis comme précédemment, $R_5$ est un radical protecteur, $R'_3$ est un radical hydroxy et $R'_4$ un atome d'hydrogène si l'on veut obtenir un dérivé de l'isoindole monohalogéné, ou bien $R'_3$ et $R'_4$ forment ensemble un radical oxo si l'on veut obtenir un dérivé de l'isoindole dihalogéné, puis élimination du radical protecteur $R_5$.

Le radical protecteur $R_5$ peut être tout groupement protecteur d'amino qui soit compatible avec la réaction

et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloacétyle, trifluoracétyle, vinyloxycarbonyle phénoxycarbonyle, chloro-1 éthoxycarbonyle ou chlorocarbonyle.

Lorsque l'on veut obtenir un dérivé fluoré de perhydroisoindole de formule générale (III), la fluoration s'effectue dans les conditions décrites précédemment pour la fluoration d'un dérivé de formule générale (I) dans laquelle $R_3$ est hydroxy, à une température comprise entre -30 et +30°C. Il est entendu que le dérivé fluoré de configuration (R) est obtenu à partir de l'alcool de configuration (S) et que le dérivé fluoré de configuration (S) est obtenu à partir du dérivé hydroxylé de configuration (R). Il est également possible d'opérer à partir d'un mélange des alcools de configuration (R) et (S) et d'effectuer la séparation au niveau du dérivé de formule générale (III).

Lorsque l'on veut obtenir le dérivé difluoré de formule générale (III), la réaction s'effectue à partir de l'isoindolone de formule générale (VII) ($R'_3$ et $R'_4$ forment ensemble un radical oxo), en opérant dans les conditions définies ci-dessus, à une température comprise entre 30°C et la température de reflux du mélange réactionnel.

Lorsque l'on veut obtenir un dérivé chloré du perhydroisoindole de formule générale (III) la chloration s'effectue selon les conditions décrites par R. J. Cremlyn et coll., J. Chem. Soc., 3794 (1954), soit au moyen de pentachlorure de phosphore à partir du dérivé hydroxylé de configuration (S) lorsque l'on veut obtenir le dérivé chloré de configuration (R), soit au moyen de chlorure de thionyle à partir du dérivé hydroxylé de configuration (S) lorsque l'on veut obtenir un dérivé chloré de configuration (S). Il est entendu que l'on peut aussi effectuer la séparation au niveau du produit de formule générale (III).

Lorsque l'on veut obtenir le dérivé dichloré, on opère à partir de la perhydroisoindole de formule générale (VII), par traitement par le pentachlorure de phosphore dans les conditions citées ci-dessus.

L'élimination subséquente du radical protecteur $R_5$ s'effectue selon les méthodes habituelles. Notamment selon les méthodes décrites par T.W. Greene, par A. Wiley ou par Mc Omie dans les références citées précédemment.

Le dérivé de l'isoindole de formule générale (III) pour lequel $R_3$ est un atome d'halogène et $R_4$ est un atome d'hydrogène, peut également être obtenu par halogénation d'un dérivé de perhydroisoindole de formule générale :

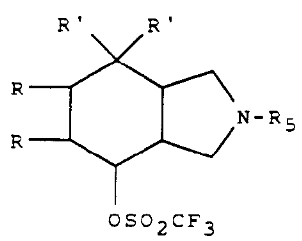

(VIIa)

dans laquelle R et R' sont définis comme précédemment, puis élimination du radical protecteur $R_5$.

L'halogénation s'effectue par un halogénure d'ammonium quaternaire tel que par exemple fluorure de tétrabutylammmonium ou par un halogénure alcalin tel que le fluorure de potassium ou le fluorure de césium par exemple, en milieu anhydre, dans un solvant organique tel qu'un éther (tétrahydrofuranne, dioxanne par exemple) un solvant chloré (dichlorométhane par exemple) ou dans un mélange de solvants, à une température comprise entre -30 et 50°C.

Il est entendu que le dérivé sulfonylé de formule générale (VIIa) de configuration (S) conduit à un dérivé halogéné de configuration (R) et que le dérivé sulfonylé de configuration (R) conduit à un dérivé halogéné de configuration (S).

L'élimination du radical $R_5$ s'effectue comme décrit précédemment.

Le dérivé sulfonylé de formule générale (VIIa) peut être obtenu par traitement du dérivé de perhydroisoindole de formule générale (VII) pour lequel $R'_3$ est un radical hydroxy et $R'_4$ est un atome d'hydrogène, par un dérivé réactif de l'acide trifluorométhane sulfonique.

La réaction s'effectue généralement par action de l'anhydride trifluorométhane sulfonique en présence de pyridine, dans un solvant chloré (dichlorométhane par exemple), à une température voisine de -30°C.

Le dérivé de perhydroisoindole de formule générale (VII) peut être préparé par protection de l'amino du dérivé correspondant de formule générale :

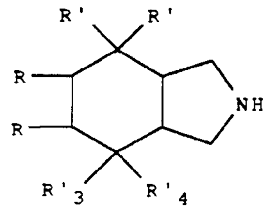

(VIII)

dans laquelle R, R′, $R'_3$ et $R'_4$ sont définis comme pour la formule générale (VII).

La protection s'effectue selon les méthodes habituelles. Notamment selon les références citées précédemment.

Le dérivé de perhydroisoindole de formule générale (VII) ou (VIII) pour lequel $R'_3$ est un radical hydroxy et $R'_4$ est un atome d'hydrogène, peut être obtenu par réduction du dérivé correspondant du perhydroisoindole de formule générale (VII) ou (VIII) pour lequel $R'_3$ et $R'_4$ forment ensemble un radical oxo.

La réduction s'effectue dans des conditions analogues à celles décrites pour l'obtention des perhydroisoindoles de formule générale (I) pour lesquels $R_3$ est hydroxy, à partir de la perhydroisoindolone correspondante.

Le dérivé hydroxylé de perhydroisoindole de formule générale (III) ou (VIII) pour lequel $R'_3$ est un radical hydroxy et $R'_4$ est un atome d'hydrogène peut être obtenu par libération du radical protecteur $R_5$ du dérivé de perhydroisoindole correspondant de formule générale (VII) dans laquelle $R'_3$ et $R'_4$ sont définis comme ci-dessus.

L'élimination s'effectue selon les méthodes connues qui n'affectent pas le reste de la molécule.

Le dérivé de l'isoindole de formule générale (VIII) pour lequel $R'_3$ et $R'_4$ forment ensemble un radical oxo, peut être obtenu à partir du dérivé correspondant de formule générale :

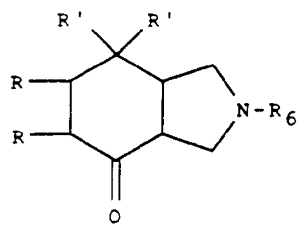

(IX)

dans laquelle R et R′ sont définis comme précédemment et $R_6$ représente un radical allyle ou un radical de structure - $CR_aR_bR_c$ dans laquelle $R_a$ et $R_b$ sont des atomes d'hydrogène ou des radicaux phényle éventuellement substitués (par un atome d'halogène, un radical alcoyle, alcoyloxy ou nitro), et $R_c$ est défini comme $R_a$ et $R_b$ ou représente un radical alcoyle ou alcoyloxyalcoyle, l'un au moins de $R_a$, $R_b$ et $R_c$ étant un radical phényle substitué ou non et les radicaux alcoyle contenant 1 à 4 atomes de carbone en chaîne droite ou ramifiée, par élimination du radical $R_6$, par toute méthode connue qui n'affecte pas le reste de la molécule.

Notamment, lorsque R est un atome d'hydrogène, et lorsque $R_5$ est autre qu'un radical allyle, le groupement $R_5$ peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque $R_5$ est un radical benzhydryle ou trityle, l'élimination peut être effectuée par traitement en milieu acide, en offrant à une température comprise entre 0°C et la température de reflux du mélange réactionnel, dans un alcool, dans un éther, dans l'eau ou directement dans l'acide acétique, l'acide formique ou l'acide trifluoroacétique.

Le groupement $R_6$ peut être également éliminé en faisant agir le chloroformiate de vinyle, le chloroformiate de chloro-1 éthyle ou le chloroformiate de phényle, en passant intermédiairement par un produit de formule générale :

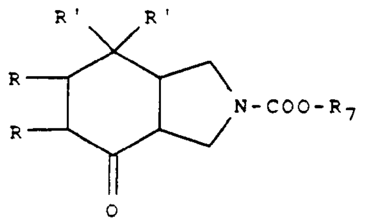

(X)

dans laquelle R et R′ sont définis comme précédemment, et $R_7$ est un radical vinyle, chloro-1 éthyle ou phényle, puis par élimination du radical $-COOR_7$ par traitement acide. L'action du chloroformiate s'effectue généralement dans un solvant organique tel qu'un solvant chloré (dichlorométhane, dichloréthane, chloroforme par exemple), un éther (tétrahydrofuranne, dioxanne par exemple) ou une cétone (acétone par exemple) ou dans un mélange de ces solvants, en opérant à une température comprise entre 20°C et la température de reflux du mélange réactionnel.

L'élimination du radical $-COOR_7$ est effectuée par traitement en milieu acide par exemple par l'acide trifloroacétique, formique, méthanesulfonique, p.toluènesulfonique, chlorhydrique ou bromhydrique dans un solvant tel qu'un alcool, un éther, un ester, un nitrile, un mélange de ces solvants ou dans l'eau, à une température comprise entre 0°C et la température de reflux du mélange réactionnel.

Dans les conditions d'élimination des radicaux $-COOR_7$ citées précédemment, le dérivé de l'isoindolone de formule générale (VIII) est obtenu à l'état de sel de l'acide employé qui peut être mis en oeuvre directement dans l'étape ultérieure.

Le dérivé de l'isoindolone de formule générale (IX) (ou (VII) lorsque $R'_3$ et $R'_4$ forment ensemble un radical oxo et $R_5$ est un radical benzyle éventuellement substitué), peuvent être obtenus par réaction de cycloaddition par action d'un dérivé silylé de formule générale :

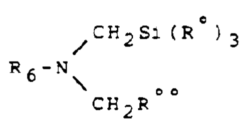

(XI)

dans laquelle $R_6$ est défini comme précédemment, $(R°)_3$ représente des radicaux alcoyle ou des radicaux alcoyle et phényle et R°° représente un radical alcoyloxy, cyano ou phénylthio, sur le dérivé de la cyclohexènone de formule générale :

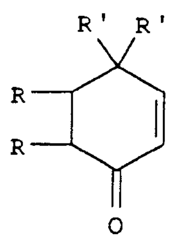

(XII)

dans laquelle R et R′ sont définis comme précédemment.

On opère en présence d'une quantité catalytique d'un acide choisi parmi l'acide trifluoroacétique, l'acide acétique, l'acide méthanesulfonique ou les acides cités dans les références mentionnées ci-dessous, dans un solvant organique tel qu'un solvant chloré (dichlorométhane, dichloréthane par exemple), dans un hydrocarbure aromatique, dans un nitrile (acétonitrile) ou dans un éther, à une température comprise entre 0°C et la température de reflux du mélange réactionnel.

Le dérivé silylé de formule générale (XI) peut être obtenu selon les méthodes décrites par :

- Y. Terao et coll., Chem. Pharm. Bull., 33, 2762 (1985);
- A. Hosomi et coll., Chem. Lett., 1117 (1984)
- A. Padwa et coll., Chem. Ber., 119, 813 (1986) ou
- Tetrahedron, 41, 3529 (1985).

Il est entendu que les dérivés de perhydroisoindole de formule générale (I), (III), (V), (VII), (VIIa), (VIII), (IX) et (X) présentent plusieurs formes stéréoisomères. Lorsque l'on veut obtenir un produit de formule générale (I) de forme (3aR,7aR), la séparation des formes isomères est mise en oeuvre de préférence au niveau du dérivé de formule générale (VIII) pour lequel $R'_3$ et $R'_4$ forment ensemble un radical oxo. Elle peut aussi être mise en oeuvre au niveau du dérivé de formule générale (III). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l'acide dibenzoyltartrique, puis séparation des isomères par cristallisation. L'isomère recherché est libéré de son sel en milieu basique.

La séparation des isomères axial et équatorial des dérivés hydroxylés ou des dérivés halogénés s'effectue avantageusement au niveau des produits de formule générale (VII) ou (VIII), en opérant par cristallisation et chromatographie. Il est également possible d'opérer au niveau des produits de formule générale (III) ou (I).

Les nouveaux dérivés de l'isoindole de formule générale (I) peuvent être purifiés le cas échéant par des méthodes physiques telles que la cristallisation ou la chromatographie.

Le cas échéant, les nouveaux dérivés de formule générale (I) pour lesquels les symboles $R_1$ et/ou $R_2$ contiennent des substituants amino ou alcoylamino et/ou X représente un radical NH, peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de l'isoindole de formule générale (I) peuvent aussi, le cas échéant, lorsque $R_2$ représente un radical carboxy, être transformés en sels métalliques ou en sels d'addition avec une base azotée, selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les nouveaux dérivés de l'isoindole selon la présente invention qui antagonisent les effets de la substance P peuvent trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des secrétions lachrymales.

En effet, les produits selon l'invention manifestent une affinité pour les récepteurs à substance P à des doses comprises entre 5 et 2000 nM selon la technique décrite par C.M. Lee et coll., Mol. Pharmacol., 23, 563-69 (1983).

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés se sont montrés actifs à des doses comprises entre 20 et 1000 nM.

La substance P est connue pour être impliquée dans un certain nombre de domaines pathologiques :

- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiocascular Pharmacology, 10 (suppl. 12), 5172 (1987).

Il a notamment été mis en évidence, par l'étude de plusieurs produits que les nouveaux dérivés de l'isoindole manifestent une activité analgésique dans la technique de Siegmund E. et coll., Proc. Soc. Exp. Biol. Med., 95, 729 (1957).

| Produit de formule générale (I) | DE 50 mg/kg p.o. |
|---|---|
| Exemple 1 | 3 |
| Exemple 2 | 2 |
| Exemple 7 | 1,5 |

L'étude de plusieurs dérivés de l'isoindole de formule générale (I) dans la technique de A. Saria et coll., Arch. Pharmacol.,324, 212-218 (1983) adaptée à la souris a permis de mettre en évidence un effet inhibiteur de l'augmentation de la perméabilité capillaire entraîné par le septide (agoniste de la substance P, ce qui témoigne d'une activité anti-inflammatoire :

| Produit de formule générale (I) | $DE_{50}$ mg/kg s.c. |
|---|---|
| Exemple 1 | 0,70 |
| Exemple 2 | 0,30 |
| Exemple 7 | 0,32 |

L'injection de substance P chez l'animal provoque une hypotension. Les produits étudiés dans la technique de C.A. Maggi et coll., J. Auton. Pharmac., 7, 11-32 (1987) manifestent un effet antagoniste chez le rat vis-à-vis de cette hypotension. Notamment les produits administrés à la dose de 1 mg/kg i.v./mn, pendant 5 mn, provoquent un antagonisme de l'hypotension induite par une injection i.v. de 250 ng/kg de substance P.

| Produit de formule générale (I) | % d'inhibition de l'hypotension |
|---|---|
| Exemple 1 | 73 |
| Exemple 2 | 68 |

Par ailleurs, les dérivés de l'isoindole selon la présente invention ne présentent pas de toxicité, ils se sont montrés atoxiques chez la souris par voie sous cutanée à la dose de de 40 mg/kg ou par voie orale à la dose de 100 mg/kg.

D'un intérêt particulier sont les produits de formule générale (I) dans laquelle :

- les radicaux R sont des atomes d'hydrogène,
- les symboles R′ sont des radicaux phényle,
- le symbole X représente un atome d'oxygène, ou un radical NH,
- le symbole $R_1$ représente un radical phényle éventuellement substitué par un radical alcoyloxy pouvant être éventuellement substitué [par un radical dialcoylamino ou dialcoylamino dont les parties alcoyle forment avec l'atome d'azote auquel elles sont rattachées, un hétérocycle à 5 à 6 chaînons], ou substitué par un radical dialcoylamino dont les parties alcoyle peuvent former avec l'atome d'azote auquel elles sont rattachées, un hétérocycle tel que défini ci-dessus,
- le symbole $R_2$ représente un atome d'hydrogène ou un radical alcoyle,
- le symbole $R_3$ représente un atome de fluor ou de chlore ou un radical hydroxy et
- le symbole $R_4$ représente un atome d'hydrogène ou simultanément à $R_3$ représente un atome de fluor.

Et parmi ces produits plus spécialement intéressants sont les produits suivants :

- {{[(pyrrolidinyl-1)-3 propoxy-2]phényl}acétyl}-2 diphényl-4,4 fluoro-7 perhydroisoindole,
- diphényl-4,4 fluoro-7 [(méthoxy-2 phényl)-2 propionyl-(S)]-2 perhydroisoindole,
- [(diméthylamino-3 propoxy)-2 phényl]acétyl-2 diphényl-4,4 fluoro-7 perhydroisoindole,
- diphényl-7,7 [(méthoxy-2 phényl)-2 propionyl-(S)]-2 perhydroisoindolol-4,
- [(méthoxy-2 phényl)acétyl]-2 diphényl-4,4 fluoro-7 perhydroisoindole,

sous leurs formes stéréoisomères ainsi que leurs mélanges et le cas échéant leurs sels.

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

## EXEMPLE 1

A une solution de 0,72 g de chlorhydrate de diphényl-4,4 fluoro-7 perhydroisoindole-(3aR,7R,7aR), 0,5 g d'acide (diméthylamino-3 propoxy)-2 phénylacétique, 0,03 g d'hydroxy-1 benzotriazole dans 75 $cm^3$ de dichlorométhane refroidie à + 4°C on ajoute en 10 minutes une solution de 0,5 g de (dyméthylamino-3 propyl)-1 éthyl-3 carbodiimide dans 50 $cm^3$ de dichlorométhane sec puis 0,37 $cm^3$ de diisopropyléthylamine. Le mélange réactionnel est agité 3 heures à 0°C puis lavé 2 fois par 50 $cm^3$ d'eau et 2 fois par 50 $cm^3$ de solution saturée de chlorure de sodium. La phase organique est séchée sur sulfate de magnésium et concentrée à sec sous pression réduite (2,7 kPa). Le résidu est repris par 21 $cm^3$ d'acide chlorhydrique 0,1 N, 50 $cm^3$ d'éther diéthylique et 30 $cm^3$ d'eau. La phase aqueuse est séparée et lyophilisée pour donner 0,85 g de chlorhydrate de [(diméthylamino-3 propoxy)-2 phényl]acétyl-2 diphényl-4,4 fluoro-7 perhydroisoindole-(3aR,7R,7aR), sous la forme d'un lyophilisat blanc.

Spectre infra-rouge (KBr, bandes caractéristiques, $cm^{-1}$): 3060, 3030, 2960, 2890, 2800, 2200, 1635, 1605, 1495, 1460, 1445, 1250, 755, 705.

Spectre RMN du proton (DMSO-$d_6$) (à température ambiante, on observe le mélange des deux rotamères): 0,95-1,35 et 1,8-2,1 (2mt, 2x1H, $CH_2$ en 6) ; 2,6-2,8 (mt, 6H, $N(CH_3)_2$) ; 3,9 et 4,05 (2mt, 2x1H, $OCH_2$) ; 4,8 et

4,85 (2d larges, J=50, 1H, CHF) ; 6,8 à 7,5 (mt, 14H, aromatiques).

Le chlorhydrate de diphényl-4,4 fluoro-7 perhydroisoindole-(3aR,7R,7aR) peut être obtenu de la manière suivante :

Une solution de 2,25 g de t-butoxycarbonyl-2 diphényl-4,4 fluoro-7 perhydroisoindole-(3aR,7R,7aR) dans 25 $cm^3$ de dioxanne est traitée par une solution 5,8 N d'acide chlorhydrique dans le dioxanne et agitée 2 heures à 20°C puis concentrée à sec sous pression réduite (2,7 kPa). Le résidu est concrété par addition de 100 $cm^3$ d'oxyde d'isopropyle, le solide est filtré et séché pour donner 1,8 g de chlorhydrate de diphényl-4,4 fluoro-7 perhydroisoindole-(3aR,7R,7aR) sous la forme d'une poudre crème.

Spectre RMN du proton (DMSO-$d_6$): 1,0-1,35 (mt, 1H du $CH_2$en 6) ; 4,9 (d large, J=50, 1H, CHF) ; 7,1 à 7,5 (mt, 14H, aromatiques) ; 9,05 et 9,9 (2 mf, 2x1H, $NH_2^+$).

Le t-butoxycarbonyl-2 diphényl-4,4 fluoro-7 perhydroisoindole-(3aR,7R,7aR) peut être obtenu de la manière suivante :

Une solution de 4,87 g de t-butoxycarbonyl-2 diphényl-4,4 trifluorométhylsulfonyloxy-7 perhydroisoindole-(3aR,7S,7aR) dans 150 $cm^3$ de dichlorométhane sec est traitée par 22,6 $cm^3$ de solution 1M de fluorure de tétrabutylammonium dans le tétrahydrofuranne puis agitée 17 heures à 20°C et concentré à sec sous pression réduite (2,7 kPa). Le résidu est chromatographié sur une colonne de gel de silice (granulométrie 0,2-0,063 mm, diamètre 4,5 cm, hauteur 35 cm) en éluant sous une pression de 0,4 bar d'azote par un mélange de cyclohexane et d'acétate d'éthyle (75/25) et en recueillant des fractions de 20 $cm^3$. Les fractions 28 à 38 sont réunies et concentrées à sec sous pression réduite (2,7 kPa) pour donner 1,44 g de t-butoxycarbonyl-2 diphényl-4,4 fluoro-7 perhydroisoindole-(3aR,7R,7R) sous la forme de cristaux blancs fondant à 200°C, $[\alpha]_D^{20}$= - 225° (c=1, $CHCl_3$).

Le t-butoxycarbonyl-2 diphényl-4,4 trifluorométhylsulfonyloxy-7 perhydroisoindole-(3aR,7S,7aR) peut être obtenu de la manière suivante :

A une solution de 6,7 g de t-butoxycarbonyl-2 diphényl-7,7 perhydroisoindolol-4-(3aR,4S,7aR) dans 100 $cm^3$ de dichlorométhane sec refroidie à -30°C on ajoute 1,5 $cm^3$ de pyridine puis, en 10 minutes, une solution de 3,2 g d'anhydride trifluorométhanesulfonique dans 25 $cm^3$ de dichlorométhane sec. Le mélange réactionnel est agité 2 heures à -30°C puis dilué par 250 $cm^3$ d'eau et 100 $cm^3$ de dichlorométhane. La phase organique est lavée par 200 $cm^3$ de solution saturée de bicarbonate de sodium et par 200 $cm^3$ de solution saturée de chlorure de sodium puis séchée et concentrée à sec sous pression réduite (2,7 kPa) pour donner 8,6 g de t-butoxycarbonyl-2 diphényl-4,4 trifluorométhylsulfonyloxy-7 perhydroisoindole-(3aR,7S,7aR) sous la forme d'une meringue jaune utilisée telle quelle dans la suite de la synthèse.

Le t-butoxycarbonyl-2 diphényl-7,7 perhydroisoindolol-4-(3aR,4S,7aR) peut être obtenu de la manière suivante :

A une solution de 13 g de diphényl-7,7 perhydroisoindolol-4-(3aR,4S,7aR) et 0,5 g de diméthylamino-4 pyridine dans 450 $cm^3$ de dichlorométhane on ajoute 10,55 g de diterbutyldicarbonate. Après agitation durant 2 heures à 25°C, le mélange réactionnel est concentré à sec sous pression réduite (2,7 kPa) et le résidu est cristallisé dans 50 $cm^3$ d'éther éthylique. On obtient 9 g de t-butoxycarbonyl-2 diphényl-7,7 perhydroisoindolol-4-(3aR,4S,7aR) sous la forme de cristaux blancs fondant à 190°C.

Le diphényl-7,7 perhydroisoindolol-4-(3aR,4S,7aR) peut être obtenu de la manière suivante :

A une solution de 100 g de chlorhydrate de diphényl-7,7 perhydroisoindolone-4-(3aR,7aR) dans 1000 $cm^3$ de méthanol absolu refroidie à 5°C on ajoute en 90 minutes une solution de 7,18 g de borohydrure de sodium dans 500 $cm^3$ de méthanol additionnée de 20 gouttes de solution concentrée de soude. Après agitation pendant 2,5 heures entre 5 et 10°C, les cristaux formés sont essorés et repris par 900 $cm^3$ d'eau et 1000 $cm^3$ d'éther éthylique. La solution est filtrée et alcalinisée par 15 $cm^3$ de solution de soude 4N puis agitée 2 h à 5°C. Les cristaux formés sont essorés, lavés à l'éther éthylique et séchés pour donner 28,8 g de diphényl-7,7 perhydroisoindolol-4-(3aR,7S,7aR) sous la forme de cristaux blancs fondant à 205°C, $[\alpha]_D^{20}$= - 230° (c=1, $CHCl_3$).

Le chlorhydrate de diphényl-7,7 perhydroisoindolone-4-(3aR,7aR) peut être préparé de la manière suivante :

A une suspension de 200 g de chlorhydrate de diphényl-7,7 perhydroisoindolone-4 (3aRS,7aRS) dans 2000 $cm^3$ d'acétate d'éthyle, on ajoute lentement, sous agitation, 500 $cm^3$ de soude aqueuse 4N ; l'agitation est poursuivie jusqu'à dissolution du produit de départ. La solution organique est lavée par 250 $cm^3$ d'eau distillée, par 250 $cm^3$ d'une solution aqueuse saturée en chlorure de sodium, séchée sur sulfate de magnésium et filtrée. A la solution ainsi obtenue, on ajoute, sous agitation, une solution de 92,8 g d'acide L-(+) mandélique dans 1000 $cm^3$ d'acétate d'éthyle ; après 4 heures d'agitation, les cristaux obtenus sont essorés, lavés par 250 $cm^3$ d'acétate d'éthyle (2 fois) et séchés. Les cristaux sont repris par 2000 $cm^3$ d'eau distillée ; le mélange est chauffé au reflux, sous agitation, pendant 15 minutes ; les cristaux insolubles sont essorés, lavés par 100 $cm^3$ d'eau distillée (2 fois) et séchés. Ils sont recristallisés dans un mélange de 1100 $cm^3$ d'acétonitrile et de 500

$cm^3$ d'eau distillée; les cristaux obtenus sont essorés, lavés par 40 $cm^3$ d'acétonitrile (3 fois) et séchés. On obtient 80 g de (L)-mandélate de diphényl-7,7 perhydroisoindolone-4 (3aR,7aR); $[\alpha]_D^{20}$ = -164° (c=1, méthanol).

A 80 g de (L)-mandélate de diphényl-7,7 perhydroisoindolone-4 (3aR,7aR), on ajoute 400 $cm^3$ de soude aqueuse 1N et 600 $cm^3$ d'acétate d'éthyle ; le mélange est agité à température ambiante jusqu'à dissolution du produit de départ ; la solution organique est lavée par 250 $cm^3$ d'eau distillée, par 250 $cm^3$ d'une solution aqueuse saturée en chlorure de sodium, séchée sur sulfate de magnésium et filtrée ; elle est acidifiée, sous agitation, par addition de 30 $cm^3$ d'acide chlorhydrique 9N ; les cristaux obtenus sont essorés, lavés par 50 $cm^3$d'acétate d'éthyle (2 fois), par 50 $cm^3$ d'oxyde d'isopropyle et séchés. On obtient 52,3 g de chlorhydrate de diphényl-7,7 perhydroisoindolone-4 (3aR,7aR), fondant à 270°C avec décomposition; $[\alpha]_D^{20}$= -282° (c=0,5, méthanol).

Le chlorhydrate de diphényl-7,7 perhydroisoindolone-4-(3aRS,7aRS) peut être préparé selon la méthode suivante :

A 15 g de palladium sur charbon à 10%, on ajoute 150 g de benzyl-2 diphényl-7,7 perhydroisoindolone-4 (3aRS,7aRS), 1500 $cm^3$ de méthanol et 450 $cm^3$ d'acide chlorhydrique 1N ; le mélange réactionnel est hydrogéné, sous agitation, à température ambiante et sous pression atmosphérique. Après 5 heures de réaction le volume théorique d'hydrogène a été absorbé ; le mélange réactionnel est filtré, puis concentré à sec sous pression réduite (2,7 kPa) ; le résidu est cristallisé dans 200 $cm^3$ d'éthanol ; les cristaux obtenus sont essorés, lavés par 50 $cm^3$d'éthanol et séchés. On obtient 110 g de chlorhydrate de diphényl-7,7 perhydroisoindolone-4-(3aRS,7aRS) fondant à 270°C avec décomposition.

Spectre de RMN du proton : 2,03 (Mt, 1H, 1H du H en 5 ou 6) ; 2,3 (Mt, 1H, 1H du - H en 5 ou 6) ; 2,48 (DD, en partie masqué, 1H du -$CH_2$- en 1) ; 2,69 (DD, 1H, 1H du -$CH_2$- en 1); 2,8 (Mt, 2H, -$CH_2$- en 6 ou 5) ; 3,34 (DD, en partie masqué, 1H du -$CH_2$- en 3) ; 3,5 (Mt, 1H, -CH- en 3a) ; 3,82 (DD, 1H, 1H du -$CH_2$- en 3) ; 3,95 (Mt, 1H, -CH- en 7a) ; 7,15 à 7,65 (Mt, 10H, aromatiques) ; 9,43 (Mf, 2H, -$NH_2$-Cl).

Spectre Infra-rouge (KBr) bandes caractéristiques en $cm^{-1}$: 3600-3300, 3100-3000, 3000-2850, 3100-2400, 1715, 1595, 1580, 1495, 1470, 1445, 775, 750, 705.

La benzyl-2 diphényl-7,7 perhydroisoindolone-4-(3aRS,7aRS) peut être préparée de la manière suivante :

A une solution de 155 g de diphényl-4,4 cyclohexane-2 one-1 et de 202 $cm^3$ de N-butoxyméthyl N-triméthylsilylméthyl benzylamine dans 1000 $cm^3$ de dichlorométhane sec, on ajoute 5 gouttes d'acide trifluoroacétique et chauffe le mélange réactionnel au reflux pendant 45 minutes. On ajoute 50 $cm^3$ de N-butoxyméthyl N-triméthylsilylméthyl benzylamine et 3 gouttes d'acide trifluoroacétique et agite encore 45 minutes au reflux avant d'ajouter de nouveau 25 $cm^3$ de N-butoxyméthyl N-triméthylsilylméthyl benzylamine et 3 gouttes d'acide trifluoroacétique. Le mélange réactionnel est agité au reflux 45 minutes puis traité par 50 g de carbonate de potassium, filtré et concentré à sec sous pression réduite (2,7 kPa). Le résidu est dissous dans 200 $cm^3$ d'oxyde d'isopropyle et la solution refroidie à 0°C pendant 1 heure. Les cristaux sont essorés, lavés 2 fois par 15 $cm^3$ d'oxyde d'isopropyle et séchés pour donner 193 g de benzyl-2 diphényl-7,7 perhydroisoindolone-4-(3aRS,7aRS) sous la forme de cristaux blancs fondant à 132°C.

La N-butoxyméthyl N-triméthylsilylméthyl benzylamine peut être préparée selon la méthode de Y. Terao et coll., Chem. Pharm. Bull., 33, 2762 (1985).

EXEMPLE 2

A une solution refroidie à +5°C de 1 g de chlorhydrate de diphényl-4,4 fluoro-7 perhydroisoindole-(3aR,7R,7aR) et de 0,924 g d'acide {[(pyrrolidinyl-1)-3 propoxy-2]phényl}-2 acétique dans 40 $cm^3$ de dichlorométhane sec, on ajoute 0,04 g d'hydrate d'hydroxybenzotriazole, puis 0,79 g chlorhydrate de (diméthylamino-3 propyl)-1 éthyl-3 carbodiimide et 0,51 $cm^3$ de diisopropyléthylamine. Après 2,5 heures d'agitation à +5°C et 20 heures à 20°C, le mélange réactionnel est lavé 2 fois par 50 $cm^3$ d'eau, séché sur sulfate de magnésium, puis concentré à sec à 40°C sous pression réduite (2,7 kPa). Le résidu est chromatographié sur colonne de gel de silice (granulométrie 0,04-0,06 mm, diamètre 2,4 cm, hauteur 31 cm), en éluant sous une pression de 0,5 bar d'azote par un mélange d'acétate d'éthyle, d'acide acétique et d'eau (60/10/10 en volumes) et en recueillant des fractions de 25 $cm^3$. Les fractions 11 à 31 sont réunies et concentrées à sec sous pression réduite (2,7 kPa). Le résidu est repris avec 20 $cm^3$ de dichlorométhane, la solution est lavée par 20 $cm^3$ de solution aqueuse d'hydroxyde de sodium 1N, puis est séchée sur sulfate de magnésium, et concentrée à sec. Ce lavage basique est effectué une nouvelle fois. On obtient 0,68 g de {{[(pyrrolidinyl-1)-3 propoxy-2]phényl}acétyl}-2 diphényl-4,4 fluoro-7 perhydroisoindole-(3aR,7R,7aR) sous la forme d'un solide blanc.

Spectre Infra-rouge (KBr, bandes caractéristiques, $cm^{-1}$) : 3085, 3055, 3035, 2950, 2875, 2785, 1640, 1600, 1495, 1455, 1440, 1245, 750, 700.

Spectre RMN du proton (DMSO-$d_6$ + $CF_3COOD$): 1,1-1,45 (mt, 1H, 1H en 6); 1,9 (mt, 4H, 2$CH_2$ en 3 et 4

du pyrolidino); 2,27 (mt, 1H, 1H en 5); 3,77 (d, J=10, 1H, H en 1); 4,03 (mt, 2H, $OCH_2$); 4,78 (d large, J=50, 1H, CHF); 7,1 à 7,5 (mt, 14H, aromatiques).

Le chlorhydrate de l'acide {[(pyrrolidinyl-1)-3 propoxy-2]phényl} acétique peut être préparé de la façon suivante:

A 24,9 g de {[(pyrrolidinyl-1)-3 propoxy-2]phényl}acétate de méthyle on ajoute 700 $cm^3$ d'une solution d'acide chlorhydrique 6N. Après 2 heures de reflux la solution est concentrée à sec à 50°C sous pression réduite. Le résidu est ensuite repris par 100 $cm^3$ de toluène puis la solution est concentrée à sec sous pression réduite. Après un traitement identique avec du toluène, on obtient 21,9 g de chlorhydrate de l'acide {[(pyrrolidinyl-1)-3 propoxy-2]phényl} acétique, sous la forme d'un solide beige, fondant à 173°C.

Spectre RMN du proton (250 MHz, DMSO-$d_6$) : 1,8 à 2,1 (mt, 4H, -$N(CH_2\underline{CH_2})_2$; 2,15 (mt, 2H, $CH_2$-$\underline{CH_2}$-$CH_2$); 2,98 et 3,52 (2mt, 2x2H, -$N(\underline{CH_2}CH_2)_2$); 3,26 (mt, 2H, $\underline{CH_2}$-N<); 3,5 (2s, 2H, $\underline{CH_2}COO$); 4,06 (t, J=6, 2H, $OCH_2$); 6,32 (mt, 2H, aromatiques en 3 et 5); 7,23 (mt, 2H, aromatiques en 4 et 6); 11, 2 (mf, 1H, -$NH^+$<).

Le {[(pyrrolidinyl-1)-3 propoxy-2]phényl} acétate de méthyle peut être préparé de la façon suivante:

A une solution de 36 g de (bromo-3 propoxy)-2 phényl acétate de méthyle dans 400 $cm^3$ d'acétonitrile, on ajoute successivement 18,4 g de carbonate de potassium, 4,5 g d'iodure de sodium, puis 9,54 g de pyrrolidine. Le mélange réactionnel est porté au reflux pendant 5 heures puis agité à 20°C pendant 20 heures et dilué avec 800 $cm^3$ de dichlorométhane. La solution est lavée par 80 $cm^3$ d'eau puis par deux fois 80 $cm^3$ de saumure. La phase chlorométhylénique est séchée sur sulfate de sodium, concentrée à sec sous pression réduite. Le résidu est chromatographié sur une colonne de gel de silice (granulométrie 0,04-0,06 mm, diamètre 4,5 cm, hauteur 48 cm), en éluant sous une pression de 0,4 bar d'azote par un mélange d'acétate d'éthyle et de méthanol (90/10 en volumes) et en recueillant des fractions de 500 $cm^3$. Les fractions 3 à 8 sont réunies et concentrées à sec à 50°C sous pression réduite (2,7 kPa). On obtient 24,9 g de {[(pyrrolidinyl-1)-3 propoxy-2]phényl}acétate de méthyle, sous la forme d'une huile orangée.

Spectre infra-rouge ($CCl_4$, bandes caractiques, $cm^{-1}$): 3065, 3030, 2950, 2930, 2880, 2790, 1740, 1600, 1585, 1490, 1770, 1455, 1430, 1250.

Le (bromo-3 propoxy)-2 phényl acétate de méthyle peut être préparé de la façon suivante:

A une solution de 153 g d'(hydroxy-2 phényl) acétate de méthyle dans 1400 $cm^3$ d'acétonitrile, on ajoute 254,5 g de carbonate de potassium et 654 $cm^3$ de dibromo-1,3 propane. La suspension obtenue est portée au reflux pendant 20 heures, refroidie à 20°C, puis filtrée. Le filtrat est concentré à sec à 50 C sous pression réduite. Le résidu est chromatographié sur une colonne de gel de silice (granulométrie 0,04-0,06 mm, diamètre 8,8 cm, hauteur 43 cm) en éluant sous une pression d'azote de 0,5 bar par un mélange de cyclohexane et d'acétate d'éthyle (95/5 en volumes) et en recueillant des fractions de 300 $cm^3$. Les fractions 18 à 53 sont réunies et concentrées à sec sous pression réduite (2,7 kPa). On obtient 130,6 g de (bromo-3 propoxy)-2 phényl acétate de méthyle, sous la forme d'une huile orange.

Spectre RMN du proton (250 MHz, $CDCl_3$) : 2,32 (qt, J=6, 5, 2H, $CH_2$-$\underline{CH_2}$-$CH_2$); 3,26 (t, J=6,5, 2H, $CH_2Br$); 3,65 (s, 2H, $COOCH_3$); 3,7 (s, 2H, $CH_2COO$); 4,13 (t, J=6,5, 2H, $OCH_2$); 6,94 (mt, 2H, aromatiques en 3 et 5); 7,25 (mt, 2H, aromatiques en 4 et 6).

EXEMPLE 3

En opérant comme à l'exemple 9 ci-après, à partir de 0,16 g d'acide diméthylamino-2 phénylacétique et de 0,30 g de chlorhydrate de diphényl-4,4 fluoro-7 perhydroisoindole- (3aR,7S,7aR) on obtient 0,11 g de [(diméthylamino-2 phényl)acétyl]-2 diphényl-4,4 fluoro-7 perhydroisoindole- (3aR,7S,7aR) sous la forme d'une meringue blanche.

Spectre infra-rouge (KBr, bandes caractéristiques, $cm^{-1}$): 3090, 3060, 3030, 2940, 2875, 2825, 2770, 1645, 1595, 1580, 1495, 1450, 1420, 755, 730, 700.

Spectre RMN du proton (à température ambiante, on observe le mélange de deux rotamères) : 2,35 et 2,58 (2s, 6H, $N(CH_3)_2$), 4,2-4,6 (mt, 1H, CHF), 6,9-7,5 (mt, 14H, aromatiques).

Le chlorhydrate de diphényl-4,4 fluoro-7 perhydroisoindole-(3aR,7S,7aR) peut être préparé de la façon suivante:

En opérant comme à l'exemple 8 ci-après à partir de 0,5 g de tertiobutyloxycarbonyl-2 diphényl-4, 4 fluoro-7 perhydroisoindole-(3aR,7S,7aR), on obtient 0,35 g de chlorhydrate de diphényl-4,4 fluoro-7 perhydroisoindole- (3aR, 7S, 7aR) sous la forme d'un solide gris.

Spectre infra-rouge (KBr, bandes caractéristiques, $cm^{-1}$): 3420, 3090, 3050, 3025, 2970, 2920, 2800-2250, 1590, 1580, 1495, 1460, 1445, 1060, 750, 730, 700.

Le tertiobutyloxycarbonyl-2 diphényl-4,4 fluoro-7 perhydro-isoindole -(3aR,7S,7aR) peut être préparé de la façon suivante:

A une solution refroidie à +5°C de 1,0 g de tertiobutyloxycarbonyl-2 diphényl-7,7 perhydroisoindolol-4-

(3aR,4R,7aR) dans 20 $cm^3$ de dichlorométhane sec, on ajoute une solution de 0,37 $cm^3$ de trifluorothio-4 morpholine dans 10 $cm^3$ de dichlorométhane sec. Après 2 heures d'agitation à +5°C, le mélange réactionnel est lavé par 20 $cm^3$ de solution aqueuse de bicarbonate de sodium à 5% puis séché sur sulfate de magnésium et concentré à sec sous pression réduite (2,7 kPa). Le résidu est chromatographié sur une colonne de gel de silice (granulométrie 0,04 mm - 0,06 mm, diamètre 2,4 cm, hauteur 35 cm), en éluant sous une pression d'azote de 0,8 bar, par un mélange de cyclohexane et d'acétate d'éthyle (90/10 en volumes) et en recueillant des fractions de 25 $cm^3$. Les fractions 25 à 34 sont réunies et concentrées à sec. On obtient 0,27 g de tertiobutyloxycarbonyl-2 diphényl-4,4 fluoro-7 perhydroisoindole-(3aR,7S,7aR) sous la forme d'une meringue blanche.

Spectre infra-rouge (KBr, bandes caractéristiques, $cm^{-1}$): 3090, 3060, 3030, 2975, 2930, 2875, 1695, 1595, 1580, 1495, 1450, 1405, 1365, 1175, 755, 730, 700.

EXEMPLE 4

A une solution de 0,57 g de bromhydrate d'acide (pyrrolidino-2) phénylacétique dans 20 $cm^3$ de dichlorométhane sec, refroidie à 4°C, on ajoute 0,28 $cm^3$ de triéthylamine et 0,32 g de carbonyldiimidazole. On agite une heure à + 4°C, puis on ajoute une solution de 0,67 g de chlorhydrate de diphényl-4,4 fluoro-7 perhydroisoindole-(3aR,7R,7aR) dans 20 $cm^3$ de dichlorométhane sec et 0,28 $cm^3$ de triéthylamine. Le mélange réactionnel est agité à température ambiante pendant 24 heures, puis dilué par 100 $cm^3$ de dichlorométhane, lavé deux fois par 50 $cm^3$ d'eau, séché sur sulfate de magnésium, filtré et concentré à sec sous pression réduite (2,7 kPa). Le résidu est chromatographié sur colonne de gel de silice (granulométrie: 0,04-0,06 mm, diamètre 3,5 cm, hauteur 38 cm), en éluant sous une pression de 0,5 bar d'azote par un mélange de cyclohexane et d'acétate d'éthyle (70/30 en volumes) et en recueillant des fractions de 20 $cm^3$. Les fractions 26 à 54 sont réunies et concentrées à sec sous pression réduite (2,7 kPa). Le résidu est cristallisé dans un mélange d'acétonitrile et d'oxyde de diisopropyle (25/75 en volumes). On obtient 0,16 g de diphényl-4,4 fluoro-7 [(pyrrolidino-2 phényl)-acétyl]-2 perhydroisoindole-(3aR,7R,7aR) sous forme de cristaux blancs fondant à 170°C.

EXEMPLE 5

A une solution de 0,19 g d'acide (diméthylamino-2 phényl)-acétique dans 15 $cm^3$ de dichlorométhane sec, refroidie à + 4°C, on ajoute 0,17 g de carbonyldiimidazole. On agite une heure à + 4°C, puis on ajoute une solution de 0,35 g de chlorhydrate de chloro-7 diphényl-4,4 perhydroisoindole-(3aR,7R,7aR) dans 10 $cm^3$ de di-chlorométhane sec, puis une solution de 0,15 $cm^3$ de triéthylamine dans 10 $cm^3$ de dichlorométhane sec. Le mélange réactionnel est agité à température ambiante pendant 20 heures, puis dilué par 120 $cm^3$ de dichlorométhane, lavé par 80 $cm^3$ d'eau, puis par une solution aqueuse saturée en chlorure de sodium, séché sur sulfate de magnésium,filtré et concentré à sec sous pression réduite (2,7 kPa). Le résidu est chromatographié sur colonne de gel de silice (granulométrie 0,04-0,06 mm, diamètre 2 cm, hauteur 22 cm), en éluant sous une pression de 0,4 bar d'azote par un mélange d'acétate d'éthyle et de cyclohexane (75/25 en volumes) et en recueillant des fractions de 20 $cm^3$. Les fractions 6 à 9 sont réunies et concentrées à sec sous pression réduite (2,7 kPa). Le produit, obtenu sous forme de base, est converti en chlorhydrate par dissolution dans 25 $cm^3$ d'éther éthylique,puis ajout de 5 $cm^3$ d'une solution 3,2 N d'acide chlorhydrique dans l'éther éthyligue, lavage à l'éther éthylique et séchage. On obtient 0,14 g de chlorhydrate de chloro-7 [(diméthylamino-2 phényl)-acétyl]-2 diphényl-4,4 perhydroisoindole-(3aR,7R,7aR) sous la forme de cristaux blancs fondant à 190°C.

Le chlorhydrate de chloro-7 diphényl-4,4 perhydro-isoindole -(3aR,7R,7aR) peut être obtenu de la manière suivante :

Une solution de 0,4 g de chloro-7 chlorocarbonyl-2 diphényl-4,4 perhydroisoindole-(3aR,7R,7aR) dans 6 $cm^3$ d'une solution aqueuse 1N d'acide chlorhydrique et 14 $cm^3$ de tétrahydrofuranne est portée à 80°C sous agitation pendant 9 heures. Le mélange réactionnel est concentré à sec sous pression réduite (2,7 kPa). On obtient 0,35 g de chlorhydrate de chloro-7 diphényl-4,4 perhydroisoindole- (3aR,7R,7aR) sous forme de solide blanc.

Spectre infra-rouge (KBr, bandes caractéristiques, $cm^{-1}$): 3055, 3025, 3000, 2250, 1600, 1495, 1580, 1460, 1445, 1435, 760, 750, 735, 700.

Le chloro-7 chlorocarbonyl-2 diphényl-4,4 perhydroisoindole-(3aR,7R,7aR) peut être obtenu de la manière suivante :

A une solution refroidie à + 4°C de 1 g de diphényl-7,7 tert-butyloxycarbonyl-2 perhydroisoindolol-4-(3aR,4S,7aR) dans 60 $cm^3$de chloroforme, on ajoute successivement 1,3 g de carbonate de calcium, puis 2 g de pentachlorure de phosphore et on agite à température ambiante pendant 20 heures. Le mélange réactionnel est ensuite filtré, dilué par 80 $cm^3$ de chloroforme, lavé deux fois par 80 $cm^3$ d'eau, séchée sur sulfate de magnésium et concentré à sec sous pression réduite (2,7 kPa). Le résidu est chromatographié sur colonne de

gel de silice (granulométrie 0,04-0,06 mm, diamètre 2,5 cm, hauteur 34 cm), en éluant sous une pression de 0,4 bar d'azote par un mélange de cyclohexane et d'acétate d'éthyle (30/70 en volumes) et en recueillant des fractions de 20 $cm^3$. Les fractions 7 à 10 sont réunies et concentrées à sec sous pression réduite (2,7 kPa). On obtient 0,44 g de chloro-7 chlorocarbonyl-2 diphényl-4,4 perhydroisoindole-(3aR,7R,7aR) sous la forme d'un solide blanc.

Spectre infra-rouge (dilution $CCl_4$, bandes caractéristiques, $cm^{-1}$): 3090, 3065, 3035, 2930, 2855, 1745, 1600, 1585, 1495, 1450, 700.

EXEMPLE 6

A une solution de 0,43 g d'acide (diméthylamino-2 Phényl) acétique dans 15 $cm^3$ de dichlorométhane sec, refroidie à + 4°C, on ajoute 0,39 g de carbonyldiimidazole. On agite une heure à + 4°C, puis on ajoute une solution de 0,84 g de chlorhydrate de chloro-7 diphényl-4,4 perhydroisoindole-(3aR,7S,7aR) dans 10 $cm^3$ de dichlorométhane sec, puis une solution de 0,34 $cm^3$ de triéthylamine dans 10 $cm^3$ de dichlorométhane sec. Le mélange réactionnel est agité à température ambiante pondant 20 heures, puis dilué par 100 $cm^3$ de dichlorométhane, lavé par 50 $cm^3$ d'eau, puis par une solution aqueuse saturée en chlorure de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3 cm, hauteur 23 cm), en éluant sous une pression de 0,4 bar d'azote par un mélange d'acétate d'éthyle et de cyclohexane (25/75 en volumes) et en recueillant des fractions de 80 $cm^3$. La fraction 2 est concentrée à sec sous pression réduite (2,7 kPa). Le produit obtenu sous forme de base, est converti en chlorhydrate par dissolution dans 4 $cm^3$ d'acétonitrile, puis ajout de 6 $cm^3$ d'une solution 3,2 N d'acide chlorhydrique dans l'éther éthylique, lavage à l'éther isopropylique et séchage. On obtient 0,08 g de chlorhydrate de chloro-7 [(diméthylamino-2 phényl)-acétyl]-2 diphényl-4,4 perhydroisoindole-(3aR,7S,7aR) sous la forme d'un solide beige.

Spectre infra-rouge (KBr, bandes caractéristiques, $cm^{-1}$): 3055, 3025, 2950, 1635, 1490, 1460, 1440, 760, 750, 700.

Spectre RMN du proton (DMSO-$d_6$) (à 403°K on observe le mélange des deux rotamères, DMSO-$d_6$ + $CF_3COOD$, signaux principaux): 3 et 3,13 (2s, 6H, $N(CH_3)_2$) ; 4,54 et 4,63 (2mt, 1H CHCl) ; 7 à 7,8 (mt, 14H, aromatiques).

Le chlorhydrate de chloro-7 diphényl-4,4 perhydroisoindole-(3aR,7S,7aR) peut être obtenu de la manière suivante :

A une solution de 1,03 g de ter-butyloxycarbonyl-2 chloro-7 diphényl-4,4 perhydroisoindole-(3aR,7S,7aR) dans 5 $cm^3$ de dioxane on ajoute 10 $cm^3$ d'une solution 6,3 N d'acide chlorhydrique dans le dioxanne. Le mélange réactionnel est agité à température ambiante pendant 2 heures, puis concentré à sec sous pression réduite (2,7 kPa). On obtient 0,84 g de chlorhydrate de chloro-7 diphényl-4,4 perhydroisoindole-(3aR,7S,7aR) sous forme d'une solide, utilisé brut dans l'essai suivant.

Le tert-butyloxycarbonyl-2 chloro-7 diphényl-4,4 perhydro-isoindole-(3aR,7S,7aR) peut être obtenu de la manière suivante :

Une solution de 1 g de tert-butyloxycarbonyl-2 diphényl-7,7 perhydroisoindolol-4-(3aR,4S,7aR) dans 10 $cm^3$ de chlorure de thionyle est agitée 3 heures à 80°C. Le mélange réactionnel est ensuite concentré à sec sous pression réduite (2,7 kPa). On obtient 1,03 g de tert-butyloxycarbonyl-2 chloro-7 diphényl-4,4 perhydroisoindole-(3aR,7S,7aR) sous la forme d'un solide, utilisé brut dans l'essai suivant.

EXEMPLE 7

A une solution de 0,36 g d'acide (méthoxy-2 phényl)-2 propionique-(S) dans 20 $cm^3$ de dichlorométhane sec, refroidie à + 4°C, on ajoute 0,32 g de carbonyldiimidazole. On agite une heure à + 4°C, puis on ajoute une solution de 0,67 g de chlorhydrate de diphényl-4,4, fluoro-7 perhydroisoindole-(3aR,7R,7aR) dans 20 $cm^3$ de dichlorométhane sec et 0,28 $cm^3$ de triéthylamine. Le mélange réactionnel est agité à température ambiante pendant 20 heures, dilué dans 200 $cm^3$ de dichlorométhane puis lavé par 50 $cm^3$ d'eau, séché sur sulfate de magnésium, filtré et concentré à sec sous pression réduite (2,7 kPa).

Le résidu est chromatographié sur colonne de gel de silice (granulométrie 0,04-0,06 mm diamètre 3cm, hauteur 20cm), en éluant sous une pression de 0,4 bar d'azote par un mélange d'acétate d'éthyle et de cyclohexane (60/40 en volumes) et en recueillant des fractions de 20 $cm^3$. Les fractions 10 à 15 sont réunies et concentrées à sec sous pression réduite (2,7 kPa). Le résidu est cristallisé dans 0,6 $cm^3$ d'oxyde d'isopropyle. Les cristaux obtenus sont essorés, lavés par de l'oxyde d'isopropyle, puis séchés. On obtient 0,19 g de diphényl-4,4 fluoro-7 [(méthoxy-2 phényl)-2 propionyl-(S)]-2 perhydroisoindole-(3aR,7R,7aR) sous forme de cristaux blancs fondant à 195°C.

Le chlorhydrate de diphényl-4,4 fluoro-7 perhydroisoindole-(3aR, 7R, 7aR) peut être obtenu de la manière suivante :

A une solution de 3,7 g de diphényl-4,4 fluoro-7 tert-butyloxycarbonyl-2 perhydroisoindole-(3aR,7R,7aR) dans 40 $cm^3$ de dioxanne on ajoute une solution de 40 $cm^3$ de dioxanne chlorhydrique 6,3 N et on agite à température ambiante pendant 2 heures. Le mélange réactionnel est concentré à sec sous pression réduite (2,7 kPa), trituré dans de l'oxyde de diisopropyle, filtré et séché. On obtient 3,1 g de chlorhydrate de diphényl-4,4 fluoro-7 perhydroisoindole-(3aR,7R,7aR) sous forme de cristaux blancs fondant à 200°C avec décomposition.

Le diphényl-4,4 fluoro-7 tert-butyloxycarbonyl-2 perhydro-isoindole-(3aR,7R,7aR) peut être obtenu de la manière suivante :

A une solution refroidie à +5°C de 9,4 g de diphényl-7,7 tert-butyloxycarbonyl-2 perhydroisoindolol-4-(3aR,4S,7aR) dans 250 $cm^3$ de dichlorométhane sec on ajoute une solution de 3,5 $cm^3$ de trifluorure de morpholinosoufre dans 50 $cm^3$ de dichlorométhane. Le mélange réactionnel est agité 4 heures à +5°C puis dilué avec 300 $cm^3$ de dichlorométhane, lavé par 250 $cm^3$ de solution aqueuse d'hydrogénocarbonate de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3,5 cm, hauteur 42 cm , en éluant sous une pression de 0,5 bar d'azote par un mélange de cyclohexane et d'acétate d'éthyle (90/10 en volumes) et en recueillant des fractions de 120 $cm^3$. Les fractions 13 à 17 sont réunies et concentrées à sec sous pression réduite (2,7 kPa). Le résidu est cristallisé dans du cyclohexane. On obtient 2,55 g de diphényl-4,4 fluoro-7 tert-butyloxycarbonyl-2 perhydroisoindole- (3aR,7R,7aR) sous forme de cristaux blancs fondant à 202°C.

L'acide (méthoxy-2 phényl)-2 propionique-(S) peut être obtenu de la manière suivante :

L'acide (méthoxy-2 phényl)-2 propionique-(S) peut être préparé, par analogie avec les méthodes décrites par D.A Evans et coll., Tetrahedron, 44, 5525, (1988), selon le mode opératoire suivant: A une solution refroidie à +5°C de 4,1 g de méthyl-4 phényl-5 [(méthoxy-2 phényl)-2-(S)-propionyl]-3 oxazolidinone-2-(4S,5S) dans 60 $cm^3$ de tétrahydrofuranne et 30 $cm^3$ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydride 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. $[\alpha]_D^{20}$ = +84,6° (c=1 ; $CHCl_3$).

La méthyl-4 phényl-5 [(méthoxy-2 phényl)-2-(S)-propionyl]-3 oxazolidinone-2-(4S,5S)peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 $cm^3$ de tétrahydrofuranne on ajoute 19,1 g d'hexaméthyl-1,1,1,3,3,3 disilazanate de sodium, on agite 45 minutes à cette température, puis on ajoute 7,72 $cm^3$ d'iodure de méthyle. Le mélange réactionnel est ensuite agité 15 heures à température ambiante, puis dilué par de l'acétate d'éthyle, lavé par 50 $cm^3$ d'eau, puis par 50 $cm^3$ d'une solution aqueuse saturée de chlorure de sodium, séché sur sulfate de magnésium et concentré à sec sous pression réduite (2,7 kPa). Le résidu obtenu est cristallisé dans l'oxyde d'Isopropyle, essoré et séché. On obtient 4,2 g de méthyl-4 phényl-5 [(méthoxy-2 phényl)-2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1,89 d'hydrure de sodium (dispersion à 80% dans la vaseline) dans 200 $cm^3$ de tétrahydrofuranne sec on ajoute à température ambiante 9,38 g d'acide méthoxy-2 phénylacétique. On refroidit cette suspension à -30°C, on ajoute 7,77 $cm^3$ de chlorure de pivaloyle, puis on ajoute enfin une solution refroidie à -78°C obtenue en additionnant une solution de 35,27 $cm^3$ de butyllithium 1,6 M dans l'hexane à une solution refroidie à -78°C de 10 g de méthyl-4 phényl-5 oxazolidinone-2-(4S,5S) dans 200 $cm^3$ de tétrahydrofuranne sec. Le mélange réactionnel est agité 45 minutes à -30°C, puis après retour à température ambiante, on ajoute 200 $cm^3$ d'une solution aqueuse saturée de chlorure d'ammonium, puis 500 $cm^3$ d'acétate d'éthyle ; aprés décantation la phase organique est lavée deux fois par 100 $cm^3$ d'eau, puis deux fois par 100 $cm^3$ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 $cm^3$. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

EXEMPLE 8

En opérant selon le mode opératoire de l'exemple 9 ci-après à partir de 0,77 g d'acide diméthylamino-2 phénylacétique et de 1,50 g de chlorhydrate de diphényl-4,4 difluoro-7,7 perhydroisoindole-(3aRS,7aRS) on obtient 1,29 g de [(diméthylamino-2 phényl)acétyl]-2 diphényl-4,4 difluoro-7,7 perhydroisoindole-(3aRS,7aRS) sous la forme d'un solide blanc fondant à 189°C.

Le chlorhydrate de diphényl-4,4 difluoro-7,7 perhydroisoindole(3aRS,7aRS) peut être préparé de la façon suivante:

A 1,8 g de tertiobutyloxycarbonyl-2 diphényl-4,4 difluoro-7,7 perhydroisoindole-(3aRS,7aRS) on ajoute 20 $cm^3$ de dioxanne et 20 $cm^3$d'acide chlorhydrique 6,3N. Après 20 heures d'agitation à température ambiante, la suspension blanche obtenue est concentrée à sec à 40°C sous pression réduite (2,7 kPa). Le résidu est lavé avec de l'oxyde de diisopropyle, le solide obtenu essoré, puis séché. On obtient 1,51 g de chlorhydrate de diphényl-4,4 difluoro-7,7 perhydroisoindole-(3aRS,7aRS) sous la forme d'un solide blanc.

Spectre Infra-rouge (KBr, bandes caractéristiques, $cm^{-1}$): 3090, 3050, 3025, 2965, 2975, 2900, 2800-2250, 1595, 1580, 1495, 1465, 1445, 760, 730, 700.

Spectre RMN du proton (DMSO-$d_6$ + $CF_3COOD$): 1,2-1,55 et 2,12 (2mt, 2x1H, $CH_2$ en 6); 3-3,3 (mt, 1H, H en 7a); 3,58 (mt, 2H, $CH_2$ en 1); 3,76 (mt, 1H, H en 3a); 7,1 à 7,5 (mt, 10H, aromatiques).

Le tertiobutyloxycarbonyl-2 diphényl-4,4 difluoro-7,7 perhydro-isoindole-(3aRS,7aRS) peut être préparé de la façon suivante:

A une solution de 3,4 $cm^3$ de trifluorure de diéthylamino soufre dans 20 $cm^3$ de dichlorométhane sec, on ajoute une solution de 5,0 g de tertiobutyloxycarbonyl-2 diphényl-7,7 perhydroisoindolone-4-(3aRS,7aRS) dans 30 $cm^3$ de dichlorométhane sec. Après 5 heures d'agitation au reflux et 20 heures à 20°C, le mélange réactionnel est lavé par 50 $cm^3$ de solution aqueuse saturée de bicarbonate de sodium et par 50 $cm^3$ d'eau, puis séché sur sulfate de magnésium, et concentré à sec. Le résidu est chromatographié sur une colonne de gel de silice (granulométrie 0,04 - 0,06 mm, diamètre 2,8 cm, hauteur 35 cm), en éluant sous une pression d'azote de 0,8 bar par un mélange de cyclohexane et d'acétate d'éthyle (95/5 puis 90/10 en volumes) et en recueillant des fractions de 25 $cm^3$. Les fractions 24 à 52 sont réunies et concentrées à sec sous pression réduite (2,7 kPa). Le résidu est cristallisé dans de l'acétate d'éthyle et de l'oxyde de diisopropyle, les cristaux sont essorés puis séchés. On obtient 1,80 g de tertiobutyloxycarbonyl-2 diphényl-4,4 difluoro-7,7 perhydroisoindole-(3aRS,7aRS) sous la forme de cristaux blancs fondant à 162°C.

EXEMPLE 9

A une solution de 0,52 g d'acide diméthylamino-2 phénylacétique dans 20 $cm^3$ de dichlorométhane sec, on ajoute 0,49 g de N,N'-carbonyldiimidazole. On agite 30 minutes à +5°C puis on ajoute une solution de 0,93 g de chlorhydrate de diphényl-7,7 perhydroisoindolol-4-(3aR,4S,7aR) et de 0,84 $cm^3$ de triéthylamine dans 10 $cm^3$de dichlorométhane. Le mélange réactionnel est agité pendant 2 heures à +5°C puis lavé par 10 $cm^3$ d'eau, séché sur sulfate de magnésium, filtré et concentré à sec sous pression réduite (2,7 kPa). Le résidu obtenu est chromatographié sur une colonne de gel de silice (0,04 mm-0,06 mm, diamètre 2 cm, hauteur 35 cm), en éluant par de l'acétate d'éthyle et en recueillant des fractions de 30 $cm^3$. Les fractions 8 à 27 sont réunies, concentrées à sec sous pression réduite (2,7 kPa). Le résidu est cristallisé dans un mélange de 4 $cm^3$d'acétonitrile et de 20 $cm^3$ d'éther éthylique. Les cristaux sont essorés, séchés sous pression réduite (2,7 kPa). On obtient 0,70 g de [(diméthylamino-2 phényl)acétyl]-2 diphényl-7,7 perhydroisoindolol-4-(3aR,4S,7aR), sous la forme d'un solide blanc fondant à 160°C, $[\alpha]^{20}D$ = -162° (c = 0,5, méthanol).

EXEMPLE 10

En opérant selon le mode opératoire de l'exemple 9 à partir de 0,26 g d'acide diméthylamino-2 phénylacétique et de 0,50 g de chlorhydrate de diphényl-7,7 perhydroisoindolol-4-(3aR,4R,7aR), on obtient 0,21 g de [(diméthylamino-2 phényl)acétyl]-2 di-phényl-7,7 perhydroisoindolol-4-(3aR,4R,7aR), sous la forme d'un solide blanc fondant à 204°C, $[\alpha]^{20}D$ = -212° (c = 0,5, méthanol).

Le chlorhydrate de diphényl-7,7 perhydroisoindolol-4-(3aR,4R,7aR) peut être préparé par hydrogénation d'une suspension de 0,70 g de benzyl-2 diphényl-7,7 perhydroisoindolol-4-(3aR,4R,7aR) dans 30 $cm^3$ de méthanol et 2,0 $cm^3$ d'acide chlorhydrique 1N à pression atmosphérique pendant 20 heures à 20°C en présence de 0,12 g d'hydroxyde de palladium à 20% sur noir de carbone. Le mélange réactionnel est filtré, concentré à sec sous pression réduite (2,7 kPa), l'huile obtenue est concrétée avec de l'éther éthylique. La suspension est filtrée, le solide essoré et séché sous pression réduite (2,7 kPa). On obtient 0,52 g de chlorhydrate de diphényl-7,7 perhydroisoindolol-4-(3aR,4R,7aR), sous la forme d'un solide blanc fondant à 220°C (avec décomposition).

Spectre infra-rouge (bandes caractéristiques, $cm^{-1}$): 3400, 3090, 3050, 3025, 3000-2800, 1600, 1580, 1495, 1465, 985, 750, 700.

Spectre RMN du proton (DMSO-$d_6$, signaux principaux): 1,06 (t large, J=14, 1H, H en 5); 1,66 (d large, J=14, 1H, H en 5); 2,17 (d large, J=14, 1H, $CH_2$ en 6); 3,8 (s large, 1H, H en 4); 5,3 (mf, 1H, OH); 7,05 à 7,45 (mt, 10H, aromatiques); 8,4 et 9,43 (mf, 2H, $NH_2^+$).

Le benzyl-2 diphényl-7,7 perhydroisoindolol-4-(3aR,4R,7aR) peut être préparé de la façon suivante:

A une solution refroidie à 0°C de 1,3 g de benzyl-2 diphényl-7,7 perhydroisoindolone-4-(3aR,7aR) dans 6,0 $cm^3$de tetrahydrofuranne, on ajoute en 5 minutes 4,0 $cm^3$ d'une solution 1M de tri-sec-butyl borohydrure de lithium dans le tetrahydrofuranne. Le mélange réactionnel après agitation 3 heures à 0°C est à nouveau additionné de 0,5 $cm^3$ de la solution 1M de borohydrure. Après 1 heure à 0°C, puis ajout de 50 $cm^3$ d'eau et 50 $cm^3$ d'acétate d'éthyle, la phase organique est décantée, lavée par 20 $cm^3$ d'eau, séchée sur sulfate de magnésium et concentrée à sec sous pression réduite (2,7 kPa). L'huile obtenue est cristallisée dans 30 $cm^3$ d'oxyde de diisopropyle, les cristaux sont essorés, séchés sous pression réduite (2,7 kPa). On obtient 0,70 g de benzyl-2 diphényl-7,7 perhydroisoindolol-4-(3aR,4R,7aR), sous la forme de cristaux blancs fondant à 154°C.

Le benzyl-2 diphényl-7,7 perhydroisoindolone-4-(3aR,7aR) peut être préparé de la façon suivante:

A une solution refroidie à 0°C de 21,7 g de chlorhydrate de diphényl-7,7 perhydroisoindolone-4-(3aR,7aR), dans 300 $cm^3$ de di-chlorométhane, et 18,5 $cm^3$ de triéthylamine, on ajoute 7,9 $cm^3$ de bromure de benzyle. Le mélange réactionnel après agitation 1 heure à 0°C et 2 heures à 20°C est lavé par 50 $cm^3$ d'eau, séché sur sulfate de magnésium, concentré à sec sous pression réduite (2,7 kPa). Le résidu est chromatographié sur une colonne de gel de silice (0,04-0,06 mm,diamètre 5 cm, hauteur 40 cm) en éluant sous une pression de 0,6 bar d'azote, par un mélange d'acétate d'éthyle et de cyclohexane (75/25 en volumes) et en recueillant des fractions de 250 $cm^3$. Les fractions 3 à 6 sont réunies et concentrées à sec sous pression réduite (2,7 kPa). On obtient 22,1 g de benzyl-7,7 perhydroisoindolone-4-(3aR,7aR), sous la forme d'un solide blanc fondant à 124°C. $[\alpha]^{20}D$ = -279°.

EXEMPLE 11

A une solution de 0,86 g de bromhydrate d'acide (pyrrolidino-2)-phényl-acétique dans 20 $cm^3$ de dichlorométhane sec, refroidie à + 4°C, on ajoute 0,42 $cm^3$ de triéthylamine et 0,49 g de carbonyldiimidazole. On agite une heure à + 4°C, puis on ajoute une solution de 1 g de chlorhydrate de diphényl-7,7 perhydroisoindolol-4-(3aR,4S,7aR) et 0,42 $cm^3$ de triéthylamine dans 10 $cm^3$ de dichlorométhane sec. Le mélange réactionnel est agité à température ambiante pendant 24 heures, puis puis lavé deux fois par 10 $cm^3$ d'eau, puis par une solution aqueuse d'hydrogénocarbonate de sodium, séché sur sulfate de magnésium, filtré et concentré à sec sous pression réduite (2,7 kPa). Le produit, obtenu sous forme de base, est converti en chlorhydrate par dissolution dans la quantité minimale d'acétone et traitement par une solution d'acide chlorhydrique dans l'éther éthylique et ajout d'éther éthylique. Le solide obtenu est trituré dans l'éther éthylique, puis séché. On obtient 0,2 g de chlorhydrate de diphényl-7,7[(pyrrolidino-2 phényl) acétyl]-2 perhydroisoindolol-4-(3aR,4S,7aR) sous forme de solide beige.

Spectre infra-rouge (KBr, bandes caractéristiques, $cm^{-1}$): 3085, 3050, 3025, 2945, 2880, 2750, 2250, 1640, 1600, 1495, 1445, 1060, 755, 730, 700.

Spectre RMN du proton (DMSO-$d_6$): 0,92 et 1,72 (2 mt, 2x1H, $CH_2$- en 5) ; 2,17 (mt, 4H, 2 $CH_2$ en 3 et 4 du pyrrolidino) ; 7 à 7,8 (mt, 14H, aromatiques).

EXEMPLE 12

En offrant comme décrit précédemment dans l'exemple 9, à partir de 1,82 g d'acide (méthoxy-2 phényl)acétique et de 3,29 g de chlorhydrate de diphényl-7,7 perhydroisoindolol-4-(3aR,4S,7aR), on obtient 3,9 g de [(méthoxy-2 phényl)acétyl]-2 diphényl-7,7 perhydroisoindolol-4-(3aR,4S,7aR) sous la forme d'un solide blanc, fondant à 246°C. $[\alpha]^{20}D$ = -174° (c = 0,37; méthanol)

EXEMPLE 13

A une solution de 0,41 g d'acide (méthoxy-2 phényl)-2 propionique-(S) dans 15 $cm^3$ de dichlorométhane sec, refroidie à + 4°C, on ajoute 0,37 g de carbonyldiimidazole. On agite une heure à + 4°C, puis on ajoute une solution de 0,75 g de chlorhydrate de diphényl-7,7 perhydroisoindolol-4-(3aR,4S,7aR). Le mélange réactionnel est agité à température ambiante pendant 20 heures, puis puis lavé deux fois par 10 $cm^3$ d'eau, séché sur sulfate de magnésium, filtré et concentré à sec sous pression réduite (2,7 kPa). Le résidu est chromatographié sur colonne de gel de silice (granulométrie 0,04-0,06 mm diamètre 3,6 cm, hauteur 37 cm), en éluant sous une pression de 0,5 bar d'azote par un mélange d'acétate d'éthyle et de cyclohexane (50/50 en volumes) et en recueillant des fractions de 50 $cm^3$. Les fractions 21 à 41 sont réunies et concentrées à sec sous pression réduite (2,7 kPa). Le résidu est trituré dans de l'oxyde d'isopropyle puis séché. On obtient 0,3 g de diphényl-7,7 [(méthoxy-2 phényl)-2 propionyl-(S)]-2 perhydroisoindolol-4-(3aR,4S,7aR) sous la forme d'une meringue blanche.

Spectre infra-rouge (KBr, bandes caractéristiques, $cm^{-1}$): 3090, 3060, 3030, 2940, 2875, 2840, 1630, 1600, 1495, 1445, 1245, 1060, 755, 730, 700.

Spectre RMN du proton (DMSO-$d_6$) (à température ambiante, on observe le mélange des deux rotamères): 0,9-1,8 (mt, 2H, $CH_2$ en 5); 1,14 et 1,23 (2d, J=7, 3H, $CH_3$); 3,55 et 3,65 (2s, 3H, $OCH_3$); 3,85 et 4,23 (2mt, 1H, -COC<u>H</u>$CH_3$-); 6,8 à 7,5 (mt, 14H, aromatiques).

Le chlorhydrate de diphényl-7,7 perhydroisoindolol-4-(3aR,4S,7aR) peut être obtenu de la manière suivante :

A une solution de 2 g de diphényl-7,7 tert-butyloxycarbonyl-2 perhydroisoindolol-4-(3aR,4S,7aR) dans 20 $cm^3$ de dioxanne on ajoute une solution de 40 $cm^3$ de dioxanne chlorhydrique 6,3 N et on agite à température ambiante pendant 5 heures. Le mélange réactionnel est concentré à sec sous pression réduite (2,7 kPa), trituré dans l'acétonitrile, filtré et séché. On obtient 1,57 g de chlorhydrate de diphényl-7,7 perhydroisoindolol-4-(3aR,4S,7aR) sous forme de cristaux blancs fondant à 266°C.

Le diphényl-7,7 tert-butyloxycarbonyl-2 perhydroisoindolol-4-(3aR,4S,7aR) et le diphényl-7,7 tert-butyloxycarbonyl-2 perhydro-isoindolol-4-(3aR,4R,7aR) peuvent être obtenus de la manière suivante :

A une solution refroidie à + 4°C de 17,8 g de diphényl-7,7 tert-butyloxycarbonyl-2 perhydroisoindolone-4-(3aR,7aR) dans un litre de méthanol on ajoute goutte à goutte pendant 40 minutes une solution de 1 g de borohydrure de sodium dans 200 $cm^3$ de méthanol puis 10 gouttes de lessive de soude. Le mélange réactionnel est agité 3 heures à + 4°C, puis on ajoute 2 $cm^3$ d'une solution aqueuse d'acide chlorhydrique 0,1 N et on concentre à sec sous pression réduite (2,7 kPa). Le résidu est dissous dans 350 $cm^3$ de dichlorométhane, lavé par 100 $cm^3$ d'eau, puis par 50 $cm^3$de solution saturée de chlorure de sodium, séché sur sulfate de magnésium, concentré à sec sous pression réduite (2,7 kPa). Le résidu est cristallisé dans 40 $cm^3$ d'éther éthylique. Les cristaux obtenus sont essorés et séchés. On obtient 8,4 g de diphényl-7,7 tert-butyloxycarbonyl-2 perhydroisoindolol-4-(3aR,4S,7aR) sous la forme de cristaux blancs fondant à 190°C. Les eaux mères de cristallisation sont concentrées à sec sous pression réduite (2,7 kPa). Le résidu est chromatographié sur colonne de gel de silice (granulométrie 0,04-0,06 mm, diamètre 4 cm, hauteur 33 cm), en éluant sous une pression de 0,4 bar d'azote par un mélange de dichlorométhane et de méthanol(96/4 en volumes) et en recueillant des fractions de 20 $cm^3$. Les fractions 18 à 21 sont réunies et concentrées à sec sous pression réduite (2,7 kPa). On obtient 1,88 g de diphényl-7,7 tert-butyloxycarbonyl-2 perhydroisoindolol-4-(3aR4R,7aR) sous forme de meringue blanche. Les fractions 26 à 31 sont réunies et concentrées à sec sous pression réduite (2,7 kPa). Le résidu est cristallisé dans 5$cm^3$ d'éther éthylique. On obtient 2,88 g supplémentaires de diphényl-7,7 tert-butyloxycarbonyl-2 perhydroisoindolol-4-(3aR,4S,7aR) sous forme de cristaux blancs fondant à 190°C.

Le diphényl-7,7 tert-butyloxycarbonyl-2 perhydroisoindolone-4-(3aR,7aR) peut être obtenu de la manière suivante :

A une solution de 20 g de chlorhydrate de diphényl-7,7 perhydroisoindolone-4-(3aR,7aR) dans 100 $cm^3$ de dichlorométhane sec et 6,17 $cm^3$ de triéthylamine on ajoute successivement 0,74 g de diméthylamino-4 pyridine, puis 14,7 g de di-tert-butyl dicarbonate. Le mélange réactionnel est agité pendant 24 heures à température ambiante, puis lavé par une solution aqueuse d'acide citrique,puis par une solution aqueuse d'hydrogénocarbonate de sodium, séché sur sulfate de magnésium, filtré et concentré à sec sous pression réduite (2,7 kPa). Le résidu est cristallisé dans 90 $cm^3$ d'éther éthylique. Les cristaux sont essorés, lavés par 10 $cm^3$ d'éther éthylique puis séchés. On obtient 14,1 g de diphényl-7,7 tert-butyloxycarbonyl-2 perhydroisoindolone-4-(3aR,7aR), sous la forme de cristaux blancs fondant à 119°C.

## EXEMPLE 14

A une solution refroidie à + 10°C de 1 g de diphényl-7,7 perhydroisoindolol-4-(3aR,4S,7aR), 0,97 g d'acide (diméthylamino-3 propoxy)-2 phénylacétique, 0,05 g d'hydroxy-1 benzotriazole dans 50 $cm^3$ de dichlorométhane on ajoute 0,766 g de (diméthylamino-3 propyl)-1 éthyl-3 carbodiimide. Le mélange réactionnel est agité 90 minutes 20°C puis lavé 2 fois par 50 $cm^3$ d'eau et par 50 $cm^3$ de solution saturée de chlorure de sodium. La phase organique est séchée sur sulfate de magnésium et concentrée à sec sous pression réduite (2,7 kPa). Le résidu est chromatographié sur une colonne de gel de silice (granulométrie 0,2-0,063 mm, diamètre 2,9 cm, hauteur 23 cm) en éluant sous une pression de 0,7 bar d'azote par des mélanges de dichloro-1,2 éthane et de méthanol (1 litre à 90/10 en volumes et 1,5 litre à 70/30 en volume) et en recueillant des fractions de 25

$cm^3$. Les fractions 10 à 84 sont réunies et concentrées à sec sous pression réduite (2,7 kPa) pour donner 1,1 g de {[(diméthylamino-3 propoxy)-2 phényl]acétyl}-2 diphényl-7,7 perhydroisoindolol-4-(3aR,4S,7aR) sous la forme d'une meringue crème.

Spectre infra-rouge (KBr, bandes caractéristiques, $cm^{-1}$): 3080, 3050, 3020, 2940, 2870, 2815, 2765, 1635, 1600, 1490, 1455, 1445, 1245, 1065, 750, 730, 700.

Spectre RMN du proton (DMSO-$d_6$) à 433°K : 1,06 et 1,76 (2mt, 2x1H, $CH_2$ en 5) ; 2,27 (s, 6H, $N(CH_3)_2$) ; 3,9 (d, J=11, 1H, 1H du $CH_2$ en 3) ; 6,8 à 7,5 (mt, 14H, aromatiques)

Une solution de 100 g d'acide hydroxy-2 phénylacétique, 75 $cm^3$ d'alcool benzylique et 0,5 g d'acide paratoluène sulfonique dans 1400 $cm^3$ de toluène est chauffée au reflux pendant 2 heures en éliminant l'eau formée. Après refroidissement, traitement par 3 g de noir animal et filtration, le mélange réactionnel est concentré à 150 $cm^3$ et additionné de 300 $cm^3$ d'oxyde d'isopropyle. Les cristaux obtenus par refroidissement à 0°C sont essorés lavés et séchés pour donner 82,5 g d'hydroxy-2 phénylacétate de benzyle. A une solution 153 g de cet ester dans un mélange de 500 $cm^3$ de dibromo-1,3 propane et 2500 $cm^3$ d'acétonitrile on ajoute 174 g de carbonate de potassium et l'on chauffe le mélange 17 heures au reflux. Le mélange réactionnel est refroidi, filtré et concentré à sec sous pression réduite (2,7 kPa). Le résidu est repris par 500 $cm^3$ d'acétate d'éthyle et la phase organique est lavée par 2 fois par 400 $cm^3$ d'eau et 2 fois par 250 $cm^3$ de solution saturée de chlorure de sodium puis séchée et concentrée à sec sous pression réduite (2,7 kPa). Le résidu est chromatographié sur une colonne le gel de silice (granulométrie 0,2-0,063 mm, diamètre 9 cm, hauteur 55 cm) en éluant par un mélange de cyclohexane et d'acétate d'éthyle (95/5 en volumes) et en recueillant des fractions de 500 $cm^3$. Les fractions 12 à 18 sont réunies et concentrées à sec sous pression réduite (2,7 kPa) pour donner 90 g de (bromo-3 propoxy)-2 phénylacétate de benzyle sous la forme d'une huile jaune. Une solution de 40 g de ce produit dans 500 $cm^3$ d'acétonitrile est chauffée en autoclave avec 27 g d'iodure de sodium et 90 g de diméthylamine pondant 16 heures à 80°C. Le mélange réactionnel est refroidi, filtré et concentré à sec sous pression réduite (2,7 kPa). Le résidu est purifié par passage acide-base pour donner 29,3 g de (diméthylamino-3 propoxy)-2 phénylacétate de benzyle sous la forme d'une huile jaune. L'hydrogènation de cet ester à pression ordinaire à 40°C dans l'acétate d'éthyle en présence d'hydroxyde de palladium puis cristallisation dans l'acétate d'éthyle conduisent à 17,5 g d'acide (diméthylamino-3 propoxy)-2 phénylacétique sous la forme de cristaux blanc fondant à 98°C.

EXEMPLE 15

En opèrant comme dans l'exemple 2, mais à partir de chlorhydrate de l'acide [(isopropylamino-3 propoxy)-2 phényl]acétique et de chlorhydrate de diphényl-4,4 fluoro-7 perhydroisoindole-(3aR,7R,7aR) on obtient une huile jaune après purification par chromatographie sur colonne de gel de silice Merck (granulométrie 0,04-0,06 mm, diamètre 3 cm, hauteur 25 cm), en éluant sous une pression de 0.5 bar d'azote par un mélange de dichlorométhane et de méthanol (90/10 en volumes) et en recueillant des fractions de 20 $cm^3$. Le chlorhydrate de cette huile conduit à l'[[[(isopropylamino-1)-3 propoxy-2] phényl] acétyl]-2 diphényl-4,4 fluoro-7 perhydroisoindole-(3aR,7R,7aR), chlorhydrate sous forme d'un solide marron.

Spectre infra-rouge (KBr, bandes caractéristiques, $cm^{-1}$) : 3090+3060+3030, 2950+2885, 2860-2250, 1640, 1605+1495, 1455+1445, 1245, 750+700.

Spectre RMN du proton (250 MHz, DMSO, $\delta$ en ppm et J en Hz) : (A température ambiante, on observe le mélange des deux rotamères); 1 à 1,35 (mt, 1H, H axial du $CH_2$ en 6); 1,18 et 1,25 (2d, J=7, 6H, $CH_3$ isopropyl); 1,8 à 2,1 (mt, H du $CH_2$ en 6 et $CH_2$ en a); 2,27 (d large, J=13,5 1H, H équatorial du $CH_2$ en 5); 2,4 à 4 (mt, H du $CH_2$ en 5, $CH_2$ en 3, $CH_2$ en 1, CH en 3a, CH en 7a, N-CH); 3,02 (mt, 2H, $NCH_2$ en b); 3,3 à 3,54 (2s, 2H, $NCOCH_2Ar$); 3,9 à 4,15 (mt, 2H, OCH2); 4,76 et 4,8 (2d large, J=50, 1H , CHF); 6,7 à 7, 5 (mt, 14H, H aromatiques).

EXEMPLE 16

En opérant comme dans l'exemple 2, mais à partir de chlorhydrate de l'acide [(benzylamino-3 propoxy)-2 phényl]acétique et de chlorhydrate de diphényl-4,4 fluoro-7 perhydroisoindole-(3aR,7R,7aR) on obtient une huile jaune après purification par chromatographie sur colonne de gel de silice Merck (granulométrie 0,04-0,06 mm, diamètre 3 cm, hauteur 25 cm), en éluant sous une pression de 0,5 bar d'azote par un mélange d'acétate d'éthyle et de méthanol (95/5 en volumes) et en recueillant des fractions de 20 $cm^3$. L'oxalate acide de cette huile conduit au [[[(benzylamino-1)-3 propoxy-2] phényl] acétyl]-2 diphényl-4,4 fluoro-7 perhydroisoindole-(3aR, 7R, 7aR), oxalate acide sous forme d'un solide marron.

Spectre infra-rouge (KBr, bandes caractéristiques, $cm^{-1}$) : 3090+3060+3030, 2955+2885, 3250-2250, 1780+1720, 1640, 1625, 1605+1498, 1455+T445, 1245, 750+700.

Spectre RMN du proton (250 MHz, DMSO, δ en ppm et J en Hz) : (A température ambiante, on observe le mélange des deux rotamères); 1 à 1,4 (mt, 1H, H axial du $CH_2$ en 6); 1,8 à 4,2 (mt, autre H du $CH_2$ en 6, $CH_2$ en 5, $CH_2$ en 3, $CH_2$ en 1, CH en 3a et CH en 7a, $OCH_2$-$CH_2$-$CH_2N$); 3,27 et 3,53 (2s, 2H, $NCOCH_2Ar$); 4,1 et 4,18 (2s, 2H, $NCH_2Ar$), 4,78 et 4,81 (2dmt, J=50, 1H, CH-F); 6,7 à 7,6 (mt, 19H, aromatiques).

EXEMPLE 17

En opérant comme dans l'exemple 2, mais à partir de 1,26 g de chlorhydrate de l'acide [(diéthylamino-3 propoxy)-2 phényl]acétique et de 1,2 g de chlorhydrate de diphényl-4,4 fluoro-7 perhydroisoindole-(3aR,7R,7aR) on obtient après purification par chromatographie sur une colonne de gel de silice, 1,5 g de chlorhydrate de {[(diéthylamino-3 propoxy)-2 phényl]acétyl}-2 diphényl-4,4 fluoro-7 perhydroisoindole-(3aR,7R,7aR), sous la forme d'un solide blanc.

Spectre infra-rouge (KBr, bandes caractéristiques, $cm^{-1}$) : 3090, 3060, 3030, 2950, 2885, 2800, 2200, 1640, 1600, 1498, 1455, 1250, 1050, 750, 700.

Spectre RMN du proton (250 MHz, DMSO d6 + $CD_3COOD$, à température ambiante on observe le mélange de deux rotamères): 1 à 1,35 (mt, 1H, H-6 axial); 1,18 et 1,23 (2t, J= 6,5, 6H en totalité, -$CH_2\underline{CH_3}$); 1,8 à 2,2 (mt, 3H, H-6′ et $CH_2$-$\underline{CH_2}$-$CH_2$); 2,26 (d large, J= 13,5, 1H, H équatorial en 5); 2,4 à 4 (mt, 6H, $CH_2$ en 1, $CH_2$ en 3, H-3a et H-7a); 2,8 (td, J= 13,5 et 3, 1H, H-5 axial); 3 à 3,2 (mt, 6H, $\underline{CH_2}$-N($\underline{CH_2}CH_3)_2$); 3,3 et 3,53 (2s, 2H, =$NCO\underline{CH_2}Ar$); 3,9 à 4,1 (mt, 2H, $OCH_2$); 4,76 et 4,81 (2d larges, J= 50, 1H en totalité, =CHF); 6,7 à 7,5 (mt, 14H, aromatiques).

Le chlorhydrate de l'acide [(diéthylamino-3 propoxy)-2 phényl]acétique peut être préparé en offrant comme il est décrit dans l'exemple 20 à partir de 1,24 g de [(diéthylamino-3 propoxy)-2 phényl]acétate de méthyle et de 20 $cm^3$ d'acide chlorhydrique 6N. On obtient 1,26 g de chlorhydrate de l'acide [(diéthylamino-3 propoxy)-2 phényl]acétique, sous la forme d'un solide marron clair.

Spectre infra-rouge(KBr, bandes caractéristiques, $cm^{-1}$) : 3250-2250, 3100-3000, 3000-2850, 1722, 1600, 1585, 1495, 1472, 1450, 1385, 1250, 760.

Spectre RMN du proton (250 MHz, DMSO-$d_6$) : 1,25 (t, J= 7,5, 6H, 2x$CH_2\underline{CH_3}$) ; 2,14 (mt, 2H, $CH_2$-$\underline{CH_2}$-$CH_2$) ; 3,05 à 3, 3 (mt, 6H, $\underline{CH_2}$-N($\underline{CH_2}CH_3)_2$) ; 3,53 (2s, 2H, $\underline{CH_2}COO$) ; 4,08 (t, J=6, 2H, $OCH_2$) ; 6,33 (mt, 2H, aromatiques en 3 et 5) ; 7,23 (mt, 2H, aromatiques en 4 et 6); 10,62 (mf, 1H, -$NH^+$<) ; 12,2 (mf, 1H, COOH).

Le [(diéthylamino-3 propoxy)-2 phényl]acétate de méthyle peut être préparé comme il est décrit dans l'exemple 20, à partir de 1,8 g de (bromo-3 propoxy)-2 phényl acétate de méthyle et de 0,66 $cm^3$ de diéthylamine. On obtient après purification par chromatographie sur une colonne de gel de silice, 1,24 g de [(diéthylamino-3 propoxy)-2 phényl]acétate de méthyle, sous la forme d'une huile orange qui cristallise à température ordinaire.

Spectre infra-rouge ($CCl_4$, bandes caractéristiques, $cm^{-1}$) : 3070, 3030, 2970, 2930, 2875, 2800, 1740, 1605, 1590, 1495, 1475, 1455, 1435, 1250.

Spectre RMN du proton (250 MHz, $CDCl_3$) : 1,10 (t, J= 7,5, 6H, 2x$CH_2\underline{CH_3}$) ; 1, 96 (mt, 2H, $CH_2$-$\underline{CH_2}$-$CH_2$) ; 2, 62 (q, J=7, 4H, N($\underline{CH_2}CH_3)_2$) ; 2,68 (t, J=7, 2H, $\underline{CH_2}$-$NEt_2$) ; 3,64 (2s, 2H, $\underline{CH_2}COO$) ; 3,7 (s, 3H, $COOCH_3$) ; 4,03 (t, J=6, 2H, $OCH_2$) ; 6,9 (mt, 2H, aromatiques en 3 et 5), 7, 22 (mt, 2H, aromatiques en 4 et 6).

EXEMPLE 18

En opérant comme dans l'exemple 2, mais à partir de 1,37 g de l'acide {[N-(hydroxy-2 éthyl) N-méthylamino]-3 propoxy-2}phénylacétique et de 1,2 g de chlorhydrate de diphényl-4,4 fluoro-7 perhydroisoindole-(3aR,7R,7aR) on obtient après purification par chromatographie sur une colonne de gel de silice, 0,86 g de {{[N-(hydroxy-2 éthyl) N-méthylamino]-3 propoxy-2}phénylacétyl}-2 diphényl-4,4 fluoro-7 perhydroisoindole-(3aR,7R,7aR), sous la forme d'une meringue blanche.

Spectre infra-rouge(KBr, bandes caractéristiques, $cm^{-1}$) : 3425, 3105, 3090, 3060, 3030, 2950, 2885, 2850, 2800, 1640, 1600, 1495, 1455, 1445, 1250, 1050, 755, 700.

Spectre RMN du proton (400 MHz, DMSO-$d_6$ + $CD_3COOD$ d4, à 303°K) : (A cette température on observe un mélange de rotamères); 1,14 (dt large, J=46 et 13,5, 1H, H-6 axial) ; 1,85 (mt, 1H, H-6 équatorial) ; 2,03 (mt, 2H, $CH_2$-$\underline{CH_2}$-$CH_2$) ; 2,20 (mt, 1H, H-5) ; 2,3 à 3,8 (mt, H-5′, $CH_2$-1 et $CH_2$-3, H-7a et H-3a, >$NCOCH_2Ar$, -$CH_2$-N-$CH_2CH_2O$-) ; 2,75 et 2,80 (2s, 3H, >N-$CH_3$) ; 3,8 à 4 (mt, 2H, $ArOCH_2$) ; 4,7 et 4,74 (2 dmt, J=50, 1H, CHF) ; 6,7 à 7,4 (mt, 14H, aromatiques).

L'acide {[N-(hydroxy-2 éthyl) N-méthylamino]-3 propoxy-2} phénylacétique peut être préparé en opérant comme décrit dans l'exemple 20 à partir de 2,1 g de {{[N-(hydroxy-2 éthyl) N-méthylamino]-3 propoxy-2}phényl}acétate de méthyle et de 30 $cm^3$ d'acide chlorhydrique 6N. On obtient 2,27 g d'acide {[N-(hydroxy-2 éthyl) N-méthylamino]-3 propoxy-2}phénylacétique, sous la forme d'une huile rouge.

Spectre infra-rouge (entre lamelles, bandes caractéristiques, $cm^{-1}$): 1725, 1605, 1590, 1498, 1475, 1455, 1465, 1250, 1055, 760.

Spectre RMN du proton (400 MHz, DMSO-$d_6$, 383°K): 1,98 (qt, J=6,5, 2H, $CH_2$-$\underline{CH_2}$-$CH_2$) ; 2,84 (s, 3H, >N-$CH_3$) ; 3,15 à 3,4 (mt, 4H, NCH2) ; 3,56 (s, 2H, $CH_2COO$) ; 3,84 (t, J=6, $\underline{CH_2}$OH) ; 4,1 (t, J=6,5, Ar$OCH_2$); 6,96 (mt, 2H, aromatiques en 3 et 5) ; 7,24 (mt, 2H, aromatiques en 4 et 6).

Le {{[N-(hydroxy-2 éthyl) N-méthylamino]-3 propoxy-2}phényl}acétate de méthyle peut être préparé comme il est décrit dans l'exemple 20, à partir de 1,8 g de (bromo-3 propoxy)-2 phényl acétate de méthyle et de 0,5 $cm^3$ de (méthylamino)-2 éthanol. On obtient après purification par chromatographie sur une colonne de gel de silice, 0,5 g de {[N-(hydroxy-2 éthyl) N-méthylamino]-3 propoxy-2}phényl acétate de méthyle, sous la forme d'une huile rosâtre.

Spectre infra-rouge ($CH_2Cl_2$, bandes caractéristiques, $cm^{-1}$) : 3460, 3050, 3030, 3000-2850, 2810, 1738, 1600, 1590, 1498, 1455, 1435, 1250.

Spectre RMN du proton (250 MHz, $CDCl_3$) : 2,07 (mt, 2H, $CH_2$-$\underline{CH_2}$-$CH_2$) ; 2,2 à 2,8 (mf, 1H, OH) ; 2,43 (s, 3H, >N-$CH_3$) ; 2,72 (t, J=5,5, 2H, N$\underline{CH_2}$CH2OH) ; 2,76 (t, J=7, 2H, $CH_2N$) ; 3,64 (s, 2H, $CH_2COO$) ; 3,71 (s, 3H, $CO_2CH_3$) ; 3,71 (t, J=5,5, $\underline{CH_2}$OH) ; 4,04 (t, J=6, Ar$OCH_2$) ; 6,32 (mt, 2H, aromatiques en 3 et 5) ; 7,24 (mt, 2H, aromatiques en 4 et 6).

EXEMPLE 19

En opérant comme dans l'exemple 2, mais à partir de 1,5 g de chlorhydrate de l'acide [(diisopropylamino-3 propoxy)-2 phényl] acétique et de 1,3 g de chlorhydrate de diphényl-4,4 fluoro-7 perhydroisoindole-(3aR,7R,7aR) on obtient après purification par chromatographie sur colonne de gel de silice 1,57 g de chlorhydrate de {[(diisopropylamino-3 propoxy)-2 phényl]acétyl}-2 diphényl-4,4 fluoro-7 perhydroisoindole-(3aR,7R,7aR), sous la forme d'un solide blanc.

Spectre infra-rouge(KBr, bandes caractéristiques, $cm^{-1}$) : 3090, 3060, 3030, 2950, 2855, 2800-2200, 1640, 1605, 1495, 1455, 1445, 1247, 755, 700.

Spectre RMN du proton (250 MHz, DMSO-$d_6$ + $CD_3COOD$, à température ambiante on observe le mélange de deux rotamères) : 1 à 1,4 (mt, 1H, H-6 axial) ; 1,2 à 1,4 (mt, 12H, -$CH_3$ isopropyles); 1,8 à 2,2 (mt, 3H, H-6' et $CH_2$-$\underline{CH_2}$-$CH_2$) ; 2,25 (d large, J=13,5, 1H, H-5 équatorial) ; 2,4 à 4 (mt, H-5', $CH_2$-3, $CH_2$-1, H-3a, H-7a et -N($\underline{CH}(CH_3)_2)_2$) ; 3,15 (mt, 2H, -$CH_2N$<) ; 3,3 et 3,55 (2s, 2H, >NCO$CH_2$Ar) ; 3,9 à 4,1 (mt, 2H, $OCH_2$) ; 4,75 et 4,79 (2d, J=50, 1H, CHF); 6,7 à 7,5 (mt, 13H, aromatiques).

Le chlorhydrate de l'acide [(diisopropylamino-3 propoxy)-2 phényl] acétique peut être préparé en offrant comme il est décrit dans l'exemple 20 à partir de 1,50 g de [(diisopropylamino-3 propoxy)-2 phényl] acétate de méthyle et de 30 $cm^3$ d'acide chlohydrique 6N. On obtient 1,5g de chlorhydrate de l'acide [(diisopropylamino-3 propoxy)-2 phényl] acétique, sous la forme d'une huile marron.

Spectre infra-rouge (KBr, bandes caractéristiques, $cm^{-1}$) : 3500-2250, 3100-3000, 3000-2850, 1727, 1605, 1595, 1500, 1470, 1455, 1425, 1250, 760.

Spectre RMN du proton (250 MHz, DMSO-$d_6$) : 1,31 et 1,36 (2d, J=6,5, 12H, -$CH_3$ isopropyles); 2,2 (mt, 2H, $CH_2$-$\underline{CH_2}$-$CH_2$) ; 3,21 (mt, J=7, 2H, -$CH_2N$<) ; 3,53 (s, 2H, >NCO$CH_2$Ar) ; 3,61 (mt, 2H, -N($\underline{CH}(CH_3)_2)_2$) ; 4,07 (t, J=6, 2H, $OCH_2$) ; 6,91 (mt, 2H, aromatiques en 3 et 5) ; 7,22 (mt, 2H, aromatiques en 4 et 6); 9,98 (mf, 1H, -$NH^+$<).

Le [(diisopropylamino-3 propoxy)-2 phényl] acétate de méthyle peut être préparé comme il est décrit dans l'exemple 20, à partir de 2,65 g de (bromo-3 propoxy)-2 phényl acétate de méthyle et de 1,98 $cm^3$de diisopropylamine. On obtient 1,31 g de [(diisopropylamino-3 propoxy)-2 phényl] acétate de méthyle, sous la forme d'une huile jaune.

Spectre infra-rouge ($CCl_4$, bandes caractéristiques, $cm^{-1}$) : 3070, 3050, 3030, 2970, 2930, 2875, 1743, 1605, 1590, 1495, 1470, 1455, 1435, 1385, 1360, 1250.

Spectre RMN du proton (250 MHz, DMSO-$d_6$): 0,97 (d, J=7, 12H, -$CH_3$ isopropyles); 1,72 (quintuplet, J=7, 2H, $CH_2$-$\underline{CH_2}$-$CH_2$) ; 2, 55 (t, J=7, 2H, -$CH_2N$<) ; 2,97 (mt, 2H, -N($\underline{CH}Me)_2$) ; 3,59 et 3,62 (2s, 5H, >NCO$CH_2$Ar et $CO_2CH_3$) ; 3,97 (t, J=7, 2H, $OCH_2$) ; 6,32 (mt, 2H, aromatiques en 3 et 5) ; 7,22 (mt, 2H, aromatiques en 4 et 6).

EXEMPLE 20

En opérant comme dans l'exemple 2, mais à partir de 1,45 g de chlorhydrate de l'acide [(pipéridino-3 propoxy)-2 phényl] acétique et de 1,30 g de chlorhydrate de diphényl-4,4 fluoro-7 perhydroisoindole-(3aR,7R,7aR) on obtient après purification par chromatographie sur colonne de gel de silice 1,44 g de chlorhydrate de {[(pipéridino-3 propoxy)-2 phényl]acétyl}-2 diphényl-4,4 fluoro-7 perhydroisoindole-(3aR,7R,7aR), sous la forme

d'un solide blanc.

Spectre infra-rouge(KBr, bandes caractéristiques, $cm^{-1}$) : 3090, 3060, 3030, 2955, 2885, 2800-2200, 1640, 1605, 1498, 1455, 1445, 1250, 755, 700.

Spectre RMN du proton (250 MHz, DMSO-$d_6$ à 433°K) : 1,27 (dt large, J= 46 et 13,5, H-6 axial); 1,5 à 2,1 (mf, 7H, $N(CH_2\underline{CH_2})_2\underline{CH_2}$ et H-6'); 2,23 (mf, 2H, $CH_2$-$\underline{CH_2}$-$CH_2$); 2,32 (d large, J= 13,5, 1H, H-5 équatorial) ; 2,64 (td, J= 13,5 et 3, 1H, H-5 axial); 2,7 à 4 (mt, $CH_2$-1, $CH_2$-3, H-3a, H-7a et $N(\underline{CH_2}CH_2)_2CH_2$); 3,13 (t, J=7,5, 2H, $-CH_2N$<); 3,79 (AB, 2H, $NCOCH_2Ar$); 4,08 (mf, 2H, $OCH_2$); 4,82 (d large, J= 50, 1H, CHF); 6,8 à 7,5 (mt, 14H, aromatiques).

Le chlorhydrate de l'acide [(pipéridino-3 propoxy)-2 phényl] acétique peut être préparé de la façon suivante:

A 2,0 g de [(pipéridino-3 propoxy)-2 phényl] acétate de méthyle on ajoute une solution d'acide chlorhydrique 6N. Après 3 heures de reflux la solution est concentrée à sec à 50°C sous pression réduite. Le résidu est ensuite repris par 30 $cm^3$ de toluène puis la solution est concentrée à sec. Après un nouveau traitement avec du toluène, on obtient 1,9 g de chlorhydrate de l'acide [(pipéridino-3 propoxy)-2 phényl] acétique sous la forme d'un solide blanc, utilisé tel quel à l'étape suivante.

Le [(pipéridino-3 propoxy)-2 phényl] acétate de méthyle peut être préparé de la façon suivante:

A une solution de 2,87 g de (bromo-3 propoxy)-2 phényl acétate de méthyle dans 30 $cm^3$ d'acétonitrile, on ajoute successivement 1,51 g de carbonate de potassium, 0,3 g d'iodure de sodium, puis 1,0 $cm^3$ de pipéridine. Le mélange réactionnel est porté au reflux pendant 2 heures puis filtré. Le filtrat est concentré à sec et le résidu obtenu est chromatographié sur colonne de gel de silice (granulométrie 0,04 -0,06 mm, diamètre 4 cm, hauteur 50 cm), en éluant sous une pression de 0,1 bar d'azote par un mélange d'acétate d'éthyle et de méthanol (80/20 en volumes) et en recueillant des fractions de 125 $cm^3$. Les fractions 9 à 16 sont réunies et concentrées à sec. Le résidu est trituré dans de l'oxyde de diisopropyle, la suspension est filtrée, le filtrat est concentré à sec sous pression réduite (2,7 kPa). On obtient 2,0 g de [(pipéridino-3 propoxy)-2 phényl] acétate de méthyle sous la forme d'une huile jaune, utilisée telle quelle à l'étape suivante.

## EXEMPLE 21

En opérant selon le mode opératoire de l'exemple 20 à partir de 1,65 g de chlorhydrate de diphényl-4,4 fluoro-7 perhydroisoindole-(3aR,7R,7aR) et de 1,5 g d'acide {[(hydroxyméthyl-2 pyrrolidinyl-1-(S))-3 propoxy]-2 phényl} acétique dans 160 $cm^3$ de dichlorométhane sec, on obtient 1,27 g de chlorhydrate de diphényl-4,4 fluoro-7 {{[(hydroxyméthyl-2 pyrrolidinyl-1-(S))-3 propoxy]-2 phényl}-acétyl}-2 perhydroisoindole-(3aR,7R,7aR) sous la forme d'une meringue blanche.

Spectre RMN du proton: 1,27 (dt large, J=46 et 13,5; 1H, H axial du $-CH_2-$ en 6); 1,8 à 2,4 (mt, l'autre H du $-CH_2-$ en 6 et $-CH_2-$ de la pyrrolidine); 2,3 (d large, J=13,5; 1H, H équatorial du $-CH_2-$ en 5); 2,66 (mt, l'autre H du $-CH_2-$ en 5); 2,4 à 4 (mt, $-CH_2-$ en 1, $-CH_2-$ en 3, CH en 3a, CH en 7a et $CH_2N$); 3,85 (ab limite, $NCOCH_2$); 4 à 4,3 (mt, 4H, $-CH_2-O$); 4,84 (d large, J=50, 1H, CHF); 6,8 à 7,6 (mt, 13H aromatiques).

Spectre infra-rouge(KBr): 3420, 3105-3090-3060-3030, 2955-2885, 2800-2200, 1640, 1605-1498, 1455-1445, 1250, 1065, 1050, 755-705.

L'acide [(hydroxyméthyl-2 pyrrolidinyl-1-(S))-3 propoxy]-2 phénylacétique peut être obtenu de la manière suivante :

Une suspension de 1,65 g d'[(hydroxyméthyl-2 pyrrolidinyl-1-(S))-3 propoxy]-2 phényl acétate de méthyle dans 20 $cm^3$ d'acide chlorhydrique 6N est portée au reflux pendant 3 heures. Le mélange réactionnel est concentré à sec sous pression réduite (2,7 kPa). Le résidu obtenu est trituré dans l'éther. On obtient 1,45 g d'acide [(hydroxyméthyl-2 pyrrolidinyl-1-(S))-3 propoxy]-2 phényl acétique sous la forme d'une huile brune.

Spectre RMN du proton: 1,7 à 2,3 (mt, 6H, $-CH_2-$ de la pyrrolidine); 3 à 3,3 et 3,4 à 3,7 (2mt, 5H en totalité $NCH_2$ et NCH); 3,54 (s, 2H, $CH_2COO$); 3,76 (ab dédoublé, J=12,5 et 4, 2H, $CH_2O$); 4,06 (mt, 2H, $ArOCH_2$); 6,94 (mt, 2H aromatiques en ortho et para du OR); 7,22 (mt, 2H aromatiques en méta du OR); 10,35 (mf, 1H, $NH^+Cl^-$).

L'[(hydroxyméthyl-2 pyrrolidinyl-1-(S))-3 propoxy]-2 phényl acétate de méthyle peut être obtenu de la manière suivante :

A une solution de 1,8 g de (bromo-3 propoxy)-2 phényl acétate de méthyle dans 20 $cm^3$ d'acétonitrile on ajoute successivement 0,92 g de carbonate de potassium, 0,22 g d'iodure de sodium, puis 0,63 g d'hydroxyméthyl-2 pyrrolidine-(S). Le mélange réactionnel est chauffé à 70°C pendant 5 heures, puis aprés refroidissement, dilué par 100 $cm^3$ de dichlorométhane, lavé par 100 $cm^3$ d'eau , puis par 100 $cm^3$ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm , diamètre 3 cm , hauteur 30 cm), en éluant sous une pression de 0,4 bar d'azote par un mélange d'acétate d'éthyle et de méthanol (90/10 en volumes) et en recueillant des fractions de 100 $cm^3$. Les fractions 7 à 20 sont réunies

et concentrées à sec sous pression réduite (2,7 kPa) . On obtient 1,55 g d'[(hydroxyméthyl-2 pyrrolidinyl-1-(S))-3 propoxy]-2 phényl acétate de méthyle sous la forme d'une huile.

Spectre RMN du proton : 1,5 à 2 (mt, 4H, $CH_2$ de la pyrrolidine); 1,84 (mt, 2H, $CH_2$ de la chaine propoxy) ; 2,2 et 3,08 (2mt, 1H chacun, $NCH_2$de la pyrrolidine); 2,4 et 2,35 (mt, 1H chacun, $NCH_2$); 2, 5 (mt, 1H, NCH de la pyrrolidine); 3, 22 (dd, J=11,5 et 6,5 , 1H du $CH_2O$); 3,44 (dd, J=11,5 et 4,5 , 1H, l'autre H du $CH_2O$); 3,6 (2s, 5H, $CH_2COOCH_3$); 4 (t, J=6, 2H, $ArOCH_2$); 6,92 (mt, 2H aromatiques en ortho et para du OR); 7,22 (mt, 2H aromatiques en méta du OR).

EXEMPLE 22

En opérant comme dans l'exemple 2, mais à partir de 2,3 g de chlorhydrate de l'acide {[(delta-3-pyrrolinyl-1)-3 propoxy-2]phényl} acétique et de 2,32 g de chlorhydrate de diphényl-4,4 fluoro-7 perhydroisoindale-(3aR,7R,7aR) on obtient 3,4 g de produit brut sous la forme d'une meringue blanche. Ce produit est repris par 100 $cm^3$ d'acétate d'éthyle, la solution obtenue est lavée par 40 $cm^3$ d'une solution aqueuse à 5% de bicarbonate de sodium, extraite ensuite une première fois par 30 $cm^3$puis par 20 $cm^3$ d'acide chlorhydrique 1N. Les extraits aqueux acides sonts réunis, alcalinisés par de la soude 4N et extraits à nouveau par deux fois 50 $cm^3$ d'acétate d'éthyle. La phase organique est concentrée à sec sous pression réduite (2,7 kPa), le résidu est chromatographié sur une colonne de SEPHADEX LH-20 (diamètre 3 cm, hauteur 1,4 m) en éluant avec du méthanol et en recueillant des fractions de 13 $cm^3$. Les fractions 35 à 38 sont réunies et concentrées à sec sous pression réduite (2,7 kPa). Le résidu est trituré dans de l'éther éthylique, le solide est essoré, séché sous pression réduite (2,7 kPa). On obtient 0,49 g de chlorhydrate de {{[(delta-3-pyrrolinyl-1)-3 propoxy-2]phényl} acétyl}-2 diphényl-4,4 fluoro-7 perhydroisoindole-(3aR,7R,7aR), sous la forme d'un solide blanc.

Spectre infra-rouge (KBr, bandes caractéristiques, $cm^{-1}$) : 3090, 3060, 3030, 2945, 2880, 2800-2200, 1640, 1600, 1495, 1455, 1445, 1245, 755, 700.

Spectre RMN du proton (200 MHz, DMSO-$d_6$ + $CD_3COOD$ à 413°K): 1,32 (dt large, J=46 et 13,5, 1H, H-6 axial) ; 2,05 (mt, 1H, H-6 équatorial) ; 2,15 (qt, J=7, 2H, $CH_2$-<u>$CH_2$</u>-$CH_2$) ; 2,3 (d large, J=13,5, 1H,H-5 équatorial) ; 2,67 (td, J=13,5 et 3, 1H, H-5 axial) ; 2,6 à 3,9 (mt, $CH_2$-3, $CH_2$-1, H-3a et H-7a) ; 3,35 (t, J=7, 2H, -$CH_2$N<) ; 3,8 (AB limite, 2H, >$NCOCH_2$Ar) ; 4,07 (s, 4H, -N(<u>$CH_2$</u>CH)$_2$) ; 4,10 (t, J=7, 2H, $CH_2O$) ; 4,8 (dmt, J=50, 1H, CHF) ; 5,91 (s, 2H, CH=CH) ; 6,8 à 7,5 (mt, 14H, aromatiques).

Le chlorhydrate de l'acide {[(delta-3-pyrrolinyl-1)-3 propoxy-2]phényl}acétique peut être préparé en offrant comme il est décrit dans l'exemple 20 à partir de 8 g de {[(delta-3-pyrrolinyl-1)-3 propoxy-2]phényl}acétate de méthyle et de 100 $cm^3$ d'acide chlorhydrique 6N. On obtient 4,6 g de chlorhydrate de (delta-3 pyrrolinyl-1)-3 propoxy-2]phényl}acétique, sous la forme d'un solide beige clair fondant à 140°C.

Le {[(delta-3-pyrrolinyl-1)-3 propoxy-2]phényl}acétate de méthyle peut être préparé comme il est décrit dans l'exemple 20 à partir de 15,6 g du (bromo-3 propoxy)-2 phényl acétate de méthyle et de 3,8 g de delta-3-pyrroline. On obtient après purification par chromatographie sur une colonne de gel de silice, 8,0 g de {[(delta-3 pyrrolinyl-1)-3 propoxy-2]phényl}acétate de méthyle, sous la forme d'une huile jaune orangée.

Spectre RMN du proton (300 MHz, $CDCl_3$) : 1, 98 (mt, 2H, $CH_2$-<u>$CH_2$</u>-$CH_2$); 2,81 (t, J=7, 2H, -$CH_2$N<); 3,51 (s, 4H, -N(<u>$CH_2$</u>CH)$_2$); 3,64 (s, 2H, >$NCOCH_2$Ar); 3,7 (s, 3H, $CO_2CH_3$); 4,06 (t, J=6,5, 2H, $CH_2O$); 5,8 (s, 2H, CH=CH); 6, 9 (mt, 2H, aromatiques en 3 et 5); 7,21 (mt, 2H, aromatiques en 4 et 6).

EXEMPLE 23

A une solution refroidie à 0°C de 0,4 $cm^3$ de trifluorothio-4 morpholine dans 20 $cm^3$ de dichlorométhane anhydre, on ajoute en 20 minutes une solution de 1,32 g de [(méthoxy-2 phényl)acétyl]-2 diphényl-7,7 perhydroisoindolol-4-(3aR,4S,7aR) dans 100 $cm^3$ de dichlorométhane anhydre. Après agitation 2 heures à 20°C le mélange réactionnel est lavé par 100 $cm^3$ d'une solution aqueuse à 5% P/V de bicarbonate de sodium, séché sur sulfate de magnésium, concentré à sec sous pression réduite (2,7 kPa). Le résidu est chromatographié sur colonne de gel de silice (granulométrie 0,04-0,06 mm, diamètre 2,5 cm, hauteur 30 cm), en éluant sous une pression de 0,5 bar d'azote par un mélange de cyclohexane et d'acétate d'éthyle (50/50 en volumes) et en recueillant des fractions de 30 $cm^3$. Les fractions 10 à 23 sont réunies et concentrées à sec sous pression réduite (2,7 kPa). Le résidu est lavé par de l'éther éthylique puis par de l'oxyde de diisopropyle, le solide est essoré et séché. On obtient 0,38 g de [(méthoxy-2 phényl)acétyl]-2 diphényl-4,4 fluoro-7 perhydroisoindole-(3aR,7R,7aR) sous la forme d'un solide blanc fondant à 205°C.

EXEMPLE 24

A une solution refroidie à 10°C de 0,27 $cm^3$ de trifluorothio-4 morpholine dans 10 $cm^3$ de dichlorométhane

anhydre, on ajoute sous atmosphère d'azote en 20 minutes, une solution de 0,91 g de [(diméthylamino-2 phényl)acétyl]-2 diphényl-7,7 perhydro-isoindolol-4 mélange des isomères (3aR,4S,7aR) et (3aR,4R,7aR) (isomère 3aR,4S,7aR très majoritaire) obtenu selon l'exemple 17, dans 30 $cm^3$ de dichlorométhane anhydre. Après agitation 2 heures à 20°C, le mélange réactionnel est lavé successivement par 25 $cm^3$ d'eau, 20 $cm^3$ de solution aqueuse d'hydroxyde de sodium 0,1N puis séché sur sulfate de magnésium, concentré à sec sous pression réduite (2,7 kPa). Le résidu est chromatographié sur colonne de gel de silice Merck (granulométrie 0,04-0,06 mm, diamètre 2 cm, hauteur 40 cm), en éluant sous une pression de 0,5 bar d'azote par un mélange de cyclohexane et d'acétate d'éthyle (70/30 puis 50/50 en volumes) et en recueillant des fractions de 20 $cm^3$. Les fractions 22 à 25 sont réunies et concentrées à sec sous pression réduite (2,7 kPa). Le résidu est lavé par 10 $cm^3$ d'oxyde de diisopropyle, le solide est essoré et séché. On obtient 0,12 g de [(diméthylamino-2 phényl)acétyl]-2 diphényl-4,4 fluoro-7 perhydroisoindole-(3aR,7R,7aR), sous la forme d'un solide blanc fondant à 186°C.

EXEMPLE 25

A une suspension refroidie à 5°C de 3,0 g de [(diméthylamino-2 phényl)acétyl]-2 diphényl-7,7 perhydroisoindolone-4-(3aR,7aR) dans 150 $cm^3$ de méthanol, on ajoute en 10 minutes une solution de 0,15 g de borohydrure de sodium et de 0,05 $cm^3$ de solution aqueuse de soude (à 30%) dans 30 $cm^3$ de méthanol. Après 2 heures d'agitation à 5°C, le mélange réactionnel est concentré à volume réduit (30 $cm^3$ environ) et repris par 150 $cm^3$ de dichlorométhane. La solution obtenue est lavée par 50 $cm^3$ d'eau, séchée sur sulfate de magnésium, concentrée à sec à 50°C sous pression réduite (2,7 kPa). Le résidu est cristallisé dans 10 $cm^3$ d'acétonitrile, les cristaux sont essorés, lavés avec de l'acétonitrile et séchés. On obtient 2,2 g d'un mélange de [(dimethylamino-2 phényl)acétyl]-2 diphényl-7,7 perhydroisoindolol-4-(3aR,4S,7aR) et de [(diméthyl-amino-2 phényl) acétyl]-2 diphényl-7,7 perhydroisoindolol-4- (3aR,4R,7aR), sous la forme d'un solide blanc fondant à 156°C.

EXEMPLE 26

A une suspension refroidie à +5°C de 2,0 g de diphényl-7,7 phénylacétyl-2 perhydroisoindolone-4-(3aRS,7aRS) dans 100 $cm^3$ de méthanol, on ajoute en 10 minutes une solution de 0,12 g de borhydrure de sodium et de 0,05 $cm^3$ de soude 10N dans 20 $cm^3$ de méthanol. Le mélange réactionnel est agité 2 heures à froid, puis on ajoute 0,5 $cm^3$d'acide chlorhydrique 1N, et concentre à volume réduit, à 40°C sous pression réduite (2,7 kPA). Le résidu est repris par 50 $cm^3$ d'eau et 150 $cm^3$ de dichlorométhane. Après agitation la phase organique est séchée sur sulfate de magnésium, concentrée à sec. Le solide est cristallisé dans de l'acétonitrile, les cristaux sont lavés par de l'acétonitrile et de l'oxyde de diisopropyle, essorés, puis séchés. On obtient 0,24 g de diphényl-7,7 phénylacétyl-2 perhydroisoindolol-4-(3aRS,4RS,7aRS) sous la forme de cristaux blancs fondant à 220°C.

EXEMPLE 27

Le filtrat de la cristallisation du produit obtenu à l'exemple précédent est concentré à sec. Le résidu est chromatographié sur colonne de gel de silice (granulométrie 0,04-0,06 mm, diamètre 2 cm, hauteur 35 cm), en éluant sous une pression de 0,5 bar d'azote par un mélange de dichloro-1,2 éthane et de méthanol (95/5 en volumes) et en recueillant des fractions de 50 $cm^3$. Les fractions 14 à 17 sont réunies et concentrées à sec sous pression réduite (2,7 kPa).

On obtient 0,28 g de diphényl-7,7 phénylacétyl-2 perhydroisoindolol-4-(3aRS,4SR,7aRS) sous la forme de cristaux blancs fondant à 222°C.

EXEMPLE 28

A une solution de 0,41 g de tétrafluoroborate de (méthoxy-2 phényl)-acétimidate d'éthyle dans 10 $cm^3$ de dichlorométhane sec, on ajoute une solution de 0,6 g de chlorhydrate de diphényl-4,4 fluoro-7 perhydroisoindole-(3aR,7R,7aR) et de 0,51 $cm^3$ de triéthylamine dans 10 $cm^3$ de dichlorométhane sec. Le mélange réactionnel est chauffé 3 heures au reflux. Il est ensuite traité, après retour à tempétature ambiante, par 5 $cm^3$ d'une solution aqueuse de carbonate de potassium à 10%; la phase organique est lavée par 10 $cm^3$ d'eau distillée, séchée sur sulfate de magnésium et concentrée à sec sous pression réduite (2,7 kPa). Le résidu est chromatographié sur colonne de gel de silice (granulométrie 0,04-0,06 mm, diamètre 2 cm, hauteur 20 cm), en éluant sous une pression de 0,6 bar d'azote par un mélange d'acétate d'éthyle, d'acide acétique et d'eau (15/1/1 en volumes) et en recueillant des fractions de 25 $cm^3$. Les fractions 24 à 38 sont réunies et concentrées à sec

sous pression réduite (2,7 kPa). Le résidu est repris par 40 $cm^3$ de dichlorométhane, lavé par 10 $cm^3$ de solution aqueuse saturée de carbonate de potassium, puis par 10 $cm^3$ de solution aqueuse saturée de chlorure de sodium. La phase organique est séchée sur sulfate de magnésium et concentrée à sec sous pression réduite (2,7 kPa). Le résidu est cristallisé dans l'oxyde d'isopropyle. Les cristaux sont essorés et séchés. On obtient 0,18 g de diphényl-4,4 fluoro-7 [imino-1 (méthoxy-2 phényl)-2 éthyl]-2 perhydroisoindole-(3aR,7R,7aR) sous la forme de cristaux blancs fondant à 184°C avec décomposition.

EXEMPLE 29

A une solution de 2 g de diphényl-7,7 perhydroisoindolol-4-(3aR,4S,7aR) dans 30 $cm^3$ de dichlorométhane sec, on ajoute une solution de 1,56 g de tétrafluoroborate de (méthoxy-2 phényl)-acétimidate d'éthyle et de 0,96 $cm^3$ de triéthylamine dans 20 $cm^3$ de dichlorométhane sec, puis on porte le mélange réactionnel au reflux pendant 2 heures. On ajoute ensuite 10 $cm^3$ d'une solution aqueuse à 10% de carbonate de potassium, on décante, puis on lave la phase organique par 20 $cm^3$ d'eau, on sèche sur sulfate de magnésium, on filtre et concentre à sec sous pression réduite (2,7 kPa). Le résidu est chromatographié sur colonne d'alumine (diamètre 3,6 cm, hauteur 31 cm), en éluant sous une pression de 0,1 bar d'azote par un mélange de dichlorométhane et de méthanol (95/5 en volumes) et en recueillant des fractions de 50 $cm^3$. Les fractions 5 à 30 sont réunies et concentrées à sec sous pression réduite (2,7 kPa). Le solide obtenu est lavé à l'oxyde d'isopropyle, essoré et séché. On obtient 1,4 g de diphényl-7,7 [imino-1 (méthoxy-2 phényl)-2 éthyl]-2 perhydroisoindolol-4-(3aR,4S,7aR) sous la forme de cristaux blancs, fondant à 105°C avec décomposition.

La présente invention concerne également les compositions pharmaceutiques constituées par un produit de formule générale (I) ou un sel lorsqu'ils existent, éventuellement en association avec tout autre produit pharmaceutiquement compatible, pouvant être inerte ou physiologiquement actif. Les compositions selon l'invention peuvent être utilisées par voie parentérale, orale, rectale ou topique.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

En thérapeutique humaine, les produits selon l'invention peuvent être particulièrement utiles dans le traitement des douleurs d'origine traumatique, post-chirurgicale, menstruelle, céphaliques, dans les traitements de l'anxiété, des psychoses, de la maladie de Parkinson, de la schizophrénie, de la maladie d'Alzeimer, dans les traitements myorelaxants, dans les traitements des manifestations spasmodiques, douloureuses et inflammatoires des voies digestives (colites ulcéreuses, syndrome du colon irritable, maladie de Crohn), des voies urinaires (cystites) et des voies respiratoires (asthme, rhinites) ou en gynécologie et dans les traitements des migraines. Les nouveaux dérivés de l'isoindole sont également utiles dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses et dans les troubles inflammatoires occulaires ou dentaires.

Les produits selon l'invention peuvent également trouver une application dans les traitements des troubles

cardiovasculaires tels de l'hypotension, ou dans le traitement des troubles liés à une mauvaise régulation de la croissance (nanisme, hypothrophies secondaires à des maladies chroniques de l'enfant, ostéoporose, développement de greffes).

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

Exemple A

On prépare, selon la technique habituelle, des comprimés de produit actif ayant la composition suivante :

| | |
|---|---|
| - [(pyrrolidinyl-1)-3 propoxy-2] phénylacétyl-2 diphényl-4,4 fluoro-7 perhydroisoindole-4-(3aR,7R,7aR) ........ | 25 mg |
| - amidon ........ | 83 mg |
| - silice ........ | 30 mg |
| - stéarate de magnésium ........ | 3 mg |

Exemple B

On prépare, selon la technique habituelle, des comprimés de produit actif ayant la composition suivante :

| | |
|---|---|
| - diphényl-4,4 fluoro-7 [(méthoxy-2 phényl)-2 propionyl-(S)]-2 perhydroisoindole-(3aR,7R,7aR) ........ | 25 mg |
| - amidon ........ | 83 mg |
| - silice ........ | 30 mg |
| - stéarate de magnésium ........ | 3 mg |

**Revendications**

**1 -** Nouveau dérivé de perhydroisoindole caractérisé en ce qu'il répond à la formule générale :

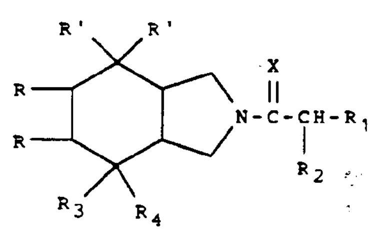

dans laquelle

- les radicaux R sont identiques et représentent des atomes d'hydrogène ou forment ensemble une liaison,
- les symboles R' sont identiques et représentent des radicaux phényle éventuellement substitués par un atome d'halogène ou par un radical méthyle en position 2 ou 3,
- le symbole X représente un atome d'oxygène, ou un radical NH,
- le symbole $R_1$ représente un radical phényle éventuellement substitué par un ou plusieurs atomes d'halogène ou radicaux hydroxy, alcoyle pouvant être éventuellement substitués (par des atomes d'halogène

ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre, et

- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,
- le symbole $R_3$ représente un atome d'halogène ou un radical hydroxy et
- le symbole $R_4$ représente un atome d'hydrogène ou simultanément à $R_3$, représente un atome d'halogène,

les radicaux alcoyle et acyle cités ci-dessus étant droits ou ramifiés et contenant 1 à 4 atomes de carbone, sous ses formes stéreoisomères ou leurs mélanges ainsi que ses sels lorsqu'ils existent.

**2 -** Un dérivé de perhydroisoindole selon la revendication 1, caractérisé en ce que

- les radicaux R sont des atomes d'hydrogène,
- les symboles R′ sont des radicaux phényle,
- le symbole X représente un atome d'oxygène, ou un radical NH,
- le symbole $R_1$ représente un radical phényle éventuellement substitué par un radical alcoyloxy pouvant être éventuellement substitué [par un radical dialcoylamino ou dialcoylamino dont les parties alcoyle forment avec l'atome d'azote auquel elles sont rattachées, un hétérocycle à 5 à 6 chaînons], ou substitué par un radical dialcoylamino dont les parties alcoyle peuvent former avec l'atome d'azote auquel elles sont rattachées, un hétérocycle tel que défini ci-dessus,
- le symbole $R_2$ représente un atome d'hydrogène ou un radical alcoyle,
- le symbole $R_3$ représente un atome de fluor ou de chlore ou un radical hydroxy et
- le symbole $R_4$ représente un atome d'hydrogène ou simultanément à $R_3$ représente un atome de fluor,

les radicaux alcoyle cités ci-dessus étant droits ou ramifiés et contenant 1 à 4 atomes de carbone, sous ses formes stéreoisomères ou leurs mélanges ainsi que ses sels lorsqu'ils existent.

**3 -** Le {{[(pyrrolidinyl-1)-3 propoxy-2]phényl}acétyl}-2 diphényl-4,4 fluoro-7 perhydroisoindole, sous ses formes stéreoisomères ou leurs mélanges ainsi que ses sels.

**4 -** Le diphényl-4,4 fluoro-7 [(méthoxy-2 phényl)-2 propionyl-(S)]-2 perhydroisoindole sous ses formes stéreoisomères ou leurs mélanges.

**5 -** Le [(diméthylamino-3 propoxy)-2 phényl]acétyl-2 diphényl-4,4 fluoro-7 perhydroisoindole sous ses formes stéreoisomères ou leurs mélanges ainsi que ses sels.

**6 -** Le diphényl-7,7 [(méthoxy-2 phényl)-2 propionyl-(S)]-2 perhydroisoindolol-4 sous ses formes stéreoisomères ou leurs mélanges.

**7 -** Le [(méthoxy-2 phényl)acétyl]-2 diphényl-4,4 fluoro-7 perhydroisoindole sous ses formes stéreoisomères ou leurs mélanges.

**8 -** Procédé de préparation d'un nouveau dérivé de perhydroisoindole selon la revendication 1, caractérisé en ce que l'on fait agir un acide ou un dérivé réactif de l'acide de formule générale

$$R_1-\underset{\displaystyle R_2}{\underset{|}{C}}H-COOH$$

dans laquelle $R_1$ et $R_2$ sont définis comme dans la revendication 1, sur un dérivé de l'isoindole de formule générale :

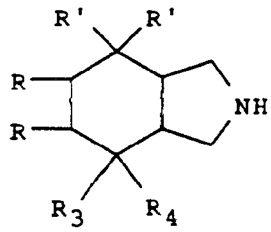

dans laquelle les symboles R, R', $R_3$ et $R_4$ sont définis comme dans la revendication 1, puis transforme éventuellement l'amide obtenu en une amidine pour laquelle X représente un radical NH, et transforme éventuellement le produit obtenu en un sel lorsqu'ils existent.

**9** - Procédé de préparation d'un dérivé perhydroisoindole selon la revendication 1, pour lequel $R_3$ est un atome d'halogène et $R_4$ est un atome d'hydrogène, caractérisé en ce que l'on fait agir un agent d'halogénation sur le dérivé correspondant selon la revendication 1, pour lequel $R_3$ est un radical hydroxy, $R_4$ est un atome d'hydrogène et X est un atome d'oxygène, puis le cas échéant transforme l'amide obtenu en une amidine et/ou en un sel lorsqu'ils existent.

**10** - Procédé de préparation d'un dérivé de perhydroisoindole selon la revendication 1 pour lequel $R_3$ est un radical hydroxy et $R_4$ est un atome d'hydrogène, caractérisé en ce que l'on réduit un dérivé de perhydroisoindolone de formule générale :

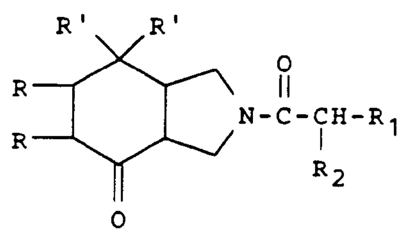

dans laquelle R, R', $R_1$ et $R_2$ sont définis comme dans la revendication 1, puis sépare éventuellement les isomères axial et equatorial et le cas échéant transforme l'amide obtenu en une amidine, et/ou en un sel lorsqu'ils existent.

**11** - Procédé de préparation d'un dérivé de l'isoindolone selon la revendication 1 pour lequel X est un radical NH et les symboles R, R', $R_1$, $R_2$ et $R_3$ sont définis comme dans la revendication 1, caractérisé en ce que l'on fait agir un produit de formule générale :

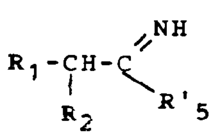

éventuellement à l'état de sel, dans laquelle $R_1$ $R_2$ sont définis comme dans la revendication 1 et $R'_5$ représente un radical alcoyloxy droit ou ramifié contenant 1 à 4 atomes de carbone ou un radical méthylthio, éthylthio, benzylthio ou alcoyloxycarbonylméthylthio, sur un dérivé de l'isoindolone de formule générale :

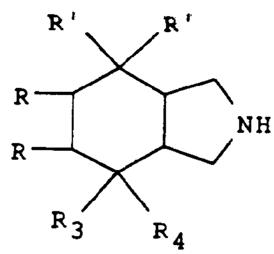

dans laquelle R, R' $R_3$ et $R_4$ définis comme dans la revendication 1, puis, lorsqu'ils existent, transforme éventuellement le produit obtenu en un sel.

**12 -** Composition pharmaceutique caractérisée en ce qu'elle contient au moins un produit selon la revendication 1, à l'état pur ou sous forme d'association avec un ou plusieurs adjuvants ou diluants compatibles et pharmaceutiquement acceptables.

Office européen des brevets

# RAPPORT DE RECHERCHE EUROPEENNE

Numero de la demande

EP 92 40 1329

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | WO-A-9 005 525 (PFIZER INC.)<br>* page 2, ligne 28 - page 4, ligne 25 *<br>--- | 1,12 | C07D209/44<br>A61K31/40 |
| P,X | EP-A-0 430 771 (RHÔNE-POULENC SANTE)<br>* page 5, ligne 58- page 6 *<br>--- | 1,12 | |
| P,X | EP-A-0 429 366 (RHÔNE-POULENC SANTE)<br>* revendications *<br>----- | 1,12 | |
| | | | DOMAINES TECHNIQUES RECHERCHES (Int. Cl.5 ) |
| | | | C07D |

Le présent rapport a été établi pour toutes les revendications

| Lieu de la recherche | Date d'achèvement de la recherche | Examinateur |
|---|---|---|
| LA HAYE | 10 AOUT 1992 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)